# EUROPEAN PATENT SPECIFICATION

(11) **EP 4 514 535 B1**
(45) Date of publication and mention of the grant of the patent: **11.03.2026**
(21) Application number: 22727760.5
(22) Date of filing: 29.04.2022
(51) Int. Cl.: B01L 3/00

(54) **ANALYSIS OF HETEROGENEOUS CELL/TISSUE SAMPLES USING A MICROFLUIDIC DEVICE**
ANALYSE HETEROGENER ZELL-/GEWEBEPROBEN MIT EINEM MIKROFLUIDISCHEN GERÄT
ANALYSE D'ÉCHANTILLONS HÉTÉROGÈNES DE CELLULES/TISSUS À L'AIDE D'UN DISPOSITIF MICROFLUIDIQUE

(43) Date of publication of application: 05.03.2025
(73) Proprietor: Technische Universität München, 80333 München (DE)
(72) Inventor: HAYDEN, Oliver, 85368 Moosburg (DE); EMKEN, Ellen, 80637 München (DE); WEIRICH, Gregor Gerhard, 81377 München (DE); BRISCHWEIN, Martin, 81371 München (DE)
(74) Representative: Lucke, Andreas
(86) International application number: PCT/EP2022/061574
(87) International publication number: WO 2023/208375

(56) References cited:
- US-A1- 2014 377 866
- US-A1- 2020 330 991
- TAVAKOLI HAMED ET AL: "Recent advances in microfluidic platforms for single-cell analysis in cancer biology, diagnosis and therapy", TRAC TRENDS IN ANALYTICAL CHEMISTRY, ELSEVIER, AMSTERDAM, NL, vol. 117, 17 May 2019 (2019-05-17), pages 13 - 26, XP085778211, ISSN: 0165-9936, [retrieved on 20190517], DOI: 10.1016/J.TRAC.2019.05.010
- MARIO ROTHBAUER ET AL: "Recent advances in microfluidic technologies for cell-to-cell interaction studies", LAB ON A CHIP, vol. 18, no. 2, 1 January 2018 (2018-01-01), UK, pages 249 - 270, XP055707408, ISSN: 1473-0197, DOI: 10.1039/C7LC00815E

## Description

### FIELD OF THE INVENTION

The present invention is in the field of biomedical research and clinical diagnostics. In particular, the invention relates to a microfluidic device for analyzing a sample comprising one or more sample objects containing biological cells, a method of analyzing a sample comprising one or more sample objects containing biological cells and a system for analyzing a sample comprising one or more sample objects containing biological cells.

### BACKGROUND

Medical diagnoses are often based on a pathological analysis of tissue and cell morphology in samples obtained from a patient. A variety of sample types may be used for this, ranging from biopsies and aspirates from solid tissues to collection of fluids with single cells and/or small cell aggregates (e.g. effusions, urine) and blood samples. The morphology of cells in a sample may for example be captured by conventional microscopy. To analyze single cells from a tissue typically requires complex and time-consuming preparation procedures. These may involve the dissociation of larger tissue chunks to be further disassembled into single cells and/or cell aggregates, see e.g. D. Soteriou et al., medRxiv 2021.11.30.21267075 and US 2011/0003324 A1, completed by a final labelling step (e.g. staining) for optimal microscopic visualization. The cell labelling may be specifically adapted to diagnostic/scientific needs (e.g. standard stains like hematoxylin and eosin (H&E) and periodic acid-Schiff (PAS), fluorescence, in situ hybridization (ISH), immunohistochemistry (IHC)). This has so far prevented high-throughput real-time analyses of such samples in a clinical setting.

An imaging technique that holds great potential for studying the morphology of single cells and cells in cell aggregates is quantitative phase-contrast microscopy and in particular digital holographic microscopy (DHM). DHM uses the interference between an imaging beam and a reference beam to obtain phase and amplitude information of light transmitted by a sample. This information can then be used to reconstruct a quantitative phase shift image of the sample, see e.g. EP 1 524 491 A1 and EP 2 357 539 A1. In recent years, digital holographic microscopes have successfully been used in biomedical applications such as live cell imaging. Phase shift images of cells may be used to reliably identify cell types based on the analysis of morphological parameters and/or using machine learning classifiers. In combination with microfluidic systems, this allows for example high-throughput label-free blood sample analyses such as blood cell counts, see e.g. US 2019/0195774 A1, facilitating the detection of diseases like malaria, leukemia, and myeloproliferative neoplasms, see for example M. Ugele et al., Adv. Sci. 1800761 (2018), WO 2019/063548 A1, and M. Ugele et al., Proc. SPIE 11060, Optical Methods for Inspection, Characterization, and Imaging of Biomaterials IV, 110600V (2019). In this way, flow cytometry can be used at the point-of-care for functional biomarker analysis based on cell types, cell morphology, cell-cell interactions, and kinetics of tissue dissociation. This solution cannot be obtained by fluorescence flow cytometry or related imaging flow cytometry methods due to the necessity of time-consuming labelling and thus the pre-defined sample volume with labeled analytes. With label-free DHM any sample volume with tissue, cells or cell aggregates can be visualized to achieve sufficient statistical power only limited by the amount of starting biopsy material. Even complex samples like solid tumors, composed of different cell types (i.e. epithelia, mesenchymal cells, and immune cells), and the tumor microenvironment are promising targets of the DHM method.

While digital holographic microscopes may be used to study cells in a liquid sample or in cell cultures including two-dimensional or three-dimensional tumor models, it is not suitable for studying larger sample objects such as pieces of continuous tissue material, e.g. from a biopsy sample. However, the latter constitute an important resource for the diagnosis and therapy of many diseases, e.g. pancreatic cancer. In the case of pancreatic lesions, for example, biopsies or fine needle aspirates are obtained by ultrasound-guided endoscopic sampling (e.g. endoscopic ultrasound-guided fine-needle biopsy (EUS-FNB) or endoscopic ultrasound-guided fine-needle aspiration (EUS-FNA)). Biopsies and fine-needle aspirates (FNA) will then be processed in histo- and cytopathology laboratories for conventional microscopic analyses to allow a morphology-based diagnosis. The post-sampling procedures are time consuming and costly and may require separate workflows. Moreover, for about 30% of patients - depending on body part or organ - surgical errors may occur as the lesion is not hit when taking the biopsy, see e.g. M. C. Omura, American Journal of Roentgenology, 197(2), 457-461 (2011). In these cases cellular material may be scarce, such that comprehensive tumor cell typing using immunohistochemistry cannot be applied, leading to inconclusive results not taking diagnostic errors into account.

US 2020 / 0 330 991 A1 discloses systems, methods, and articles of manufacture for collecting and merging two different size droplets using a substrate comprising a plurality of trapping sites. The systems may be composed of a plurality of larger droplets and smaller droplets and a substrate comprising a plurality of trapping sites where each trapping site is configured to trap only one of the larger droplets and only one of the smaller droplets when the larger droplet is already present at the trapping site. The larger and/or smaller droplets may be sorted prior to being contacted with the substrate to ensure they contain the desired component (e.g., cell or barcoded bead). Each trapping site may be composed of one or multiple fluidically linked capture wells. Collected larger and smaller droplets may be merged (e.g., via a demulsifier or electricity).

### SUMMARY OF THE INVENTION

It is thus an object of the invention to provide means for analyzing heterogeneous cell/tissue samples, which may comprise single cells, cell aggregates as well as pieces of continuous tissue material, on the single cell level, in particular using a digital holographic microscope.

This object is met by a microfluidic device for analyzing a sample comprising one or more sample objects containing biological cells according to claim 1, a method of analyzing a sample comprising one or more sample objects containing biological cells according to claim 8 and a system for analyzing a sample comprising one or more sample objects containing biological cells according to claim 13. Embodiments of the present invention are detailed in the dependent claims.

The microfluidic device according to the invention is configured for analyzing a sample comprising one or more sample objects containing biological cells, wherein the one or more sample objects comprise one or more single cells, one or more cell aggregates and/or one or more pieces of continuous tissue material. The microfluidic device comprises an insertion volume configured to receive the sample. The microfluidic device further comprises a sorting unit having an inlet that is in fluid communication with the insertion volume, a first outlet and a second outlet. The sorting unit is configured to sort the sample objects by size, weight and/or stiffness by directing sample objects having a smaller size, a smaller weight and a lower stiffness, respectively, from the inlet towards the first outlet and sample objects having a larger size, a larger weight and a higher stiffness, respectively, from the inlet towards the second outlet. The microfluidic device comprises a measurement volume that is in fluid communication with the first outlet of the sorting unit. The microfluidic device further comprises a dissociation unit comprising means for dissociating sample objects into single cells and/or cell aggregates at least in part. An inlet of the dissociation unit is in fluid communication with the second outlet of the sorting unit and an outlet of the dissociation unit is in fluid communication with an inlet of the measurement volume.

As used herein, continuous tissue material may refer to a tightly-joined structure of a large number of cells, which may e.g. comprise the cells and an extracellular matrix linking the cells to each other. A piece of continuous tissue material, which may also be referred to as a piece of tissue in the following, may for example be a biopsy sample or a small chunk of tissue from an aspiration sample. A piece of continuous tissue material may for example have a physical dimension between 0.5 mm and 2 cm in diameter and/or length and/or a volume between 10⁻³ mm³ and 8 mm³. A cell aggregate, on the other hand, may refer to a plurality of cells that are loosely bound or adhere to each other, but without forming a continuous tissue material. A cell aggregate may for example have a physical dimension between 1 µm to 500 µm in every direction and/or a volume between 3 µm³ and 10⁸ µm³. A cell aggregate may for example be a cluster of cells comprising between 2 and 100 cells, in some examples also more than 100 cells.

The microfluidic device may for example be or comprise a microfluidic chip, wherein the microfluidic chip may e.g. comprise a substrate in which the insertion volume, the sorting unit, the measurement volume, and/or the dissociation unit are formed. In some embodiments, the microfluidic device may be modular and may for example comprise a plurality of modules, some or all of which may be replaceable, e.g. to prevent cross-contamination of samples. For example, the insertion volume, the sorting unit, the measurement volume and/or the dissociation unit may each be contained in such a module, e.g. such that some (e.g. the reservoirs) or all of the aforementioned components can be replaced individually. The microfluidic device may further comprise a plurality of channels connecting the aforementioned components, for example such that the insertion volume is arranged upstream of the sorting unit and the dissociation unit and the measurement volume is arranged downstream of the sorting unit and the dissociation unit, respectively. The microfluidic device may also comprise one or more microfluidic components such as one or more pumps and/or one or more valves for generating and controlling fluid flows within the microfluidic device. The microfluidic chip may further comprise one or more reservoirs for storing fluids such as a carrier fluid in which the sample is to be dissolved or suspended, e.g. by supplying the carrier fluid to the insertion volume.

The microfluidic device may be configured to transfer or transport sample objects from a sample arranged in the insertion volume to the sorting unit, e.g. by generating a flow of a carrier fluid. The sample objects may be sorted by size, weight and/or stiffness in the sorting unit with smaller, lighter and/or less stiff sample objects being directly transferred to the measurement volume via the first outlet of the sorting unit, whereas larger and/or heavier sample objects may be transferred to the dissociation unit via the second outlet of the sorting unit for dissociating the sample objects prior to being transferred to the measurement volume. In the measurement volume, one or more measurements may be performed on the sample objects, e.g. as detailed below for the method according to the invention.

Thereby, the present invention allows for analyzing a heterogeneous sample comprising sample objects of various sizes, e.g. pieces of tissue as well as single cells and cell aggregates, and/or different types of samples such as biopsy samples and body fluid samples using the same device and similar workflows. For example, when placing a biopsy sample in the insertion volume, only blood cells (e.g. from the extraction of the biopsy sample) and/or mobile cells such as mesenchymal cells may directly pass to the measurement volume, whereas the biopsy sample itself and/or larger cell aggregates may be transferred to the dissociation unit for dissociation. The biopsy sample may be dissociated sequentially, e.g. by enzymatic digestion. This may for example allow for implementing assays to study tissue composition, e.g. by slowly dissolving the biopsy sample from the outside to the inside.

The sorting unit is configured to sort the sample objects by size, weight and/or stiffness, e.g. using a mechanical filter structure and/or by hydrodynamic and/or by inertia-based sorting as detailed below. As used herein, the size of a sample object may for example refer to a physical dimension of the respective sample object such as a length, a width, a diameter or an effective diameter or to a volume of the sample object. The sorting unit may for example be configured to sort the sample objects such that the sample objects directed towards the second outlet are larger (e.g. have a larger average size), heavier (e.g. have a larger average weight) and/or stiffer (e.g. having a higher average stiffness and/or a lower average elasticity) than the sample objects directed towards the first outlet. In some embodiments, the sorting unit may further be configured to separate the sample objects from a fluid or medium (or a part thereof) that the sample objects are contained or suspended in (e.g. a carrier fluid and/or a dissolving solution), for example to exchange said fluid or medium by a different fluid or medium (e.g. a measurement fluid). For this, the sorting unit may for example be configured to generate an accelerating force on the sample objects and the fluid/medium, e.g. similar to a centrifuge, and/or apply principles, such as lateral displacement, hydrodynamics and/or cross flow approaches. Preferably, the sorting unit is configured to sort the sample objects at least by size and/or weight (and, optionally, by stiffness in addition). In some embodiments, the sorting unit may comprise multiple sorting stages, e.g. a first sorting stage for sorting the sample objects by size and/or weight (e.g. into the first and second outlets) and a second sorting stage for sorting the sample objects by stiffness (e.g. at or downstream of the first outlet and/or the second outlet, for example to filter out dead and/or non-viable cells).

The sorting unit may for example be configured to direct sample objects having a size and/or a weight within a respective first range towards the first outlet and to direct sample objects having a size and/or a weight within a respective second range towards the second outlet, wherein an upper bound of the first range is smaller than or equal to a lower bound of the second range. In one example, the sorting unit is configured to direct sample objects having a size and/or a weight smaller/lighter than a respective sorting threshold towards the first outlet and to direct sample objects having a size and/or a weight larger/heavier than the respective sorting threshold towards the second outlet. The sorting threshold may for example be between 5 µm and 1000 µm, in some examples between 5 µm and 200 µm, in some examples between 10 µm and 100 µm, in one example between 30 µm and 70 µm. The sorting unit may for example be configured to separate single cells and small cell aggregates (e.g. cell aggregates with a size up to the sorting threshold) from larger cell aggregates and pieces of tissue. The sorting threshold may be chosen depending on the type of sample, physical dimensions of the measurement volume and/or a measurement technique used for performing measurements in the measurement volume. For a sample containing larger sample objects such as pieces of tissue (e.g. a biopsy sample), a larger sorting threshold may be used (e.g. 100 µm or 1000 µm), whereas for a sample containing smaller sample objects such as a single cells and/or small cell aggregates (e.g. a body fluid sample), a smaller sorting threshold may be used (for example 50 µm or 100 µm).

The stiffness of a sample object may for example characterize or quantify an extent to which the respective sample object resists deformation when applying a force to the sample object. The stiffness may for example be quantified by a ratio of the applied force to a displacement (e.g. a change in a physical dimension such as the length of the sample object) or by a related quantity such as an elastic modulus (e.g. Young's modulus). The sorting unit may for example be configured to sort the sample objects by stiffness based on a threshold and/or first and second ranges for the respective quantity, e.g. as detailed above for the sorting by size and/or weight. In one example, the sorting unit is configured to sort the sample objects by stiffness by directing sample objects having a Young's modulus smaller than a (stiffness) sorting threshold towards the first outlet and sample objects having a Young's modulus larger than said sorting threshold towards the second outlet, wherein said sorting threshold may for example be between 50 Pa and 30 kPa, in some examples between 210 Pa and 23 kPa, in some examples between 400 Pa and 10 kPa. In one example, said sorting threshold is between 200 Pa and 400 Pa, e.g. 300 Pa. Sorting of sample objects by stiffness may for example be implemented as described in G. Wang et al., PLOS ONE 8(10), e75901 (2013).

The dissociation unit may comprise one or more dissociation volumes, in which the sample objects are to be dissociated, e.g. dissolved chemically (for this document including chemical and/or enzymatic means) and/or dissociated mechanically (e.g. as a result of a force acting on the respective sample object). The dissociation volumes may be arranged between the inlet and the outlet of the dissociation unit. The dissociation volumes may be arranged in series, e.g. such that a first dissociation volume is arranged upstream of a second dissociation volume such that sample objects flowing through the dissociation unit first pass through the first dissociation volume and then through the second dissociation volume. Additionally or alternatively, the dissociation volumes may be arranged in parallel, e.g. such that sample objects flowing through the dissociation unit pass through either the first dissociation volume or the second dissociation volume.

The dissociation unit comprises means for dissociating sample objects into single cells and/or cell aggregates at least in part. The means for dissociating sample objects may be configured to remove single cells and/or cell aggregates from a sample object, e.g. mechanically, chemically, thermally and/or electrically, and/or to separate or break apart a sample object into two or more smaller sample objects, e.g. into smaller pieces of tissue and/or smaller cell aggregates. As used herein, mechanical dissociation may refer to any dissociation as a result of a force acting on the respective sample object, for example by cutting, grinding and/or soni-cation.

The means for dissociating sample objects may comprise a reservoir configured to supply a dissolving solution to a dissociation volume. The dissolving solution may comprise one or more chemical and/or biochemical substances for chemically dissolving sample objects. The dissolving solution may in particular be or comprise an enzymatic solution, wherein the enzymatic solution may comprise one or more enzymes for dissolving sample objects by enzymatic digestion. The one or more chemical and/or biochemical substances, e.g. the one or more enzymes, may be configured to break down macromolecules and may for example comprise one or more proteases for breaking down proteins and/or one or more nucleases for breaking down nucleic acids. The one or more chemical and/or biochemical substances/enzymes may for example be selected from the group consisting of trypsin, collagenase (e.g. collagenase type II), DNase, accutase, dispase, papain, liberase, pronase, protease, proteinase (K), thrombin and ethylenediaminetetraacetic acid (EDTA). The means for dissociating sample objects may further comprise a pump and/or a valve for supplying the dissolving/enzymatic solution from the reservoir to the dissociation volume.

Additionally or alternatively, the means for dissociating sample objects may comprise an acoustic emitter configured to apply acoustic waves (e.g. sound waves) to sample objects in a dissociation volume, in particular an ultrasound emitter configured to apply ultrasound to sample objects in a dissociation volume. The acoustic/ultrasound emitter may for example be or may comprise a passive emitter configured to transfer acoustic waves/ultrasound generated by an external acoustic/ultrasound source such as a sonotrode to the dissociation volume. A shape, a size and/or a material of the acoustic/ultrasound emitter may be adapted to increase the transmission efficiency into the dissociation volume. For example, a resonance frequency of the ultrasound emitter may be adapted to a frequency of the ultrasound to be applied to the microfluidic device and may for example be between 20 kHz and 40 kHz. Additionally or alternatively, the acoustic/ultrasound emitter may be or may comprise an active emitter configured to generate acoustic waves/ultrasound, e.g. an acoustic/ultrasonic transmitter such as a piezo element, a capacitive element and/or an inductive element that is configured to convert an electrical signal applied to the acoustic/ultrasound emitter to acoustic waves/ultrasound.

Additionally or alternatively, the means for dissociating sample objects may comprise one or more static obstacles arranged in a dissociation volume configured to mechanically dissociate sample objects colliding with the one or more obstacles. Some or all of the static obstacles may for example comprise a sharp element such as a sharp corner of the respective static obstacle or a blade that is configured to cut or slice through a sample object. In some embodiments, the static obstacles may be arranged in a one-dimensional or two-dimensional grid, for example in a grid that extends perpendicular to a direction of flow in the dissociation volume, e.g. such that the static obstacles are displaced from each other in a direction perpendicular to the direction of flow.

Additionally or alternatively, the means for dissociating sample objects may comprise one or more curved and/or constricted portions of a dissociation volume configured to dissociate sample objects by generating a shear force on sample objects flowing through the dissociation volume. A width, a height and/or a cross-sectional area of the dissociation volume perpendicular to a direction of flow through the dissociation volume may for example vary along the direction of flow, e.g. such that the dissociation volume comprises a plurality of constricted portions separating non-constricted portions having a larger width, height and/or cross-sectional area than the adjacent constricted portions. A width, a height and/or a cross-sectional area of a constricted portion may for example be less than 75%, in some examples less than 50%, in one example less than 25% of the respective quantity of an adjacent non-constricted portion upstream of the constricted portion. A width and/or a height of a constricted portion may for example be between 100 µm and 2 mm. In one example, the means for dissociating sample objects comprise a plurality of constricted portion arranged in series, wherein a width, a height and/or a cross-sectional area of the constricted portions may decrease successively along the direction of flow in some examples (e.g. such that larger constricted portions are arranged upstream of smaller constricted portions). Additionally or alternatively, the dissociation volume may for example comprise one or more meandering channels, each of which may comprise a plurality of curved portions. A radius of curvature of a sidewall of the dissociation volume in the curved and/or constricted portions and/or the physical dimensions and/or arrangement of the constricted portions may be chosen such that the shear force on sample objects flowing through the dissociation volume is sufficiently large to break up or tear apart the sample objects at least in part.

Additionally or alternatively, the means for dissociating sample objects may comprise a stirrer, particularly a magnetic stirrer, configured to move a stir bar, particularly a magnetic stir bar, in a dissociation volume for dissociating sample objects. The magnetic stirrer may for example comprise one or more inductive elements such as coils that are configured to generate a time-varying magnetic field, for example a rotating magnetic field for rotating the magnetic stir bar. The inductive elements may for example be arranged on or in a sidewall of the dissociation volume. The means for dissociating sample objects may also comprise the stir bar, which may be arranged in the dissociation volume. The stir bar may comprise a sharp element such as a blade or a sharp corner that is configured to cut or slice through a sample object.

Additionally or alternatively, the means for dissociating sample objects may comprise one or more moveable elements configured to cut and/or grind sample objects in a dissociation volume. The moveable elements may for example comprise one or more blades, which may e.g. be arranged in or on a sidewall of the dissociation volume and may be configured to be rotated in the dissociation volume and/or moved back and forth in the dissociation volume, e.g. into and out of the dissociation volume, to cut or slice through a sample object. Additionally or alternatively, the moveable elements may comprise one or more grinders, each of which may e.g. comprise one or more rotatable disks which may be equipped with protrusions such as teeth to grind a sample object.

In some embodiments, the dissociation unit comprises a plurality of dissociation volumes, each of which is associated (e.g. equipped) with a different means for dissociating sample objects. For example, the dissociation unit may comprise a first dissociation volume associated with a first means for dissociating sample objects and a second dissociation volume associated with a second means for dissociating sample objects, wherein each of the first and second means for dissociating sample objects may be embodied as detailed above. The first means may for example be a reservoir configured to supply a dissolving solution to the first dissociation volume and the second means may for example be an ultrasound emitter configured to apply ultrasound to the second dissociation volume. In some examples, the dissociation unit may comprise at least three dissociation volumes with three different means for dissociating sample objects (e.g. a reservoir for a dissolving solution, an ultrasound emitter and one or more blades), in one example four dissociation volumes with four different means for dissociating sample objects (e.g. a reservoir for a dissolving solution, an ultrasound emitter, a first set of blades and a second set of blades). The dissociation volumes may be arranged in series and/or in parallel as detailed above. In some embodiments, some or all of the dissociation volumes may be associated with more than one means for dissociating sample objects. For example, one or more of the dissociation volumes may be associated with a mechanical means and a chemical means (e.g. a reservoir for a dissolving solution) for dissociating sample objects. In one embodiment, the dissociation unit comprises a common reservoir for a dissolving solution, wherein the common reservoir is configured to supply the dissolving solution to two or more, in one example to all of the dissociation volumes, e.g. via an inlet upstream of the dissociation volumes.

In a preferred embodiment, the dissociation unit further comprises a plurality of pumps and/or valves for selectively directing sample objects into one of the dissociation volumes, e.g. one pump and/or one valve for each of the dissociation volumes. Additionally or alternatively, the sorting unit may be configured to selectively direct sample objects into one of the dissociation volumes based on a size, a weight and/or a stiffness of the respective sample object, e.g. as detailed below.

The sorting unit may comprise one or more of an inertia-based cell filter, an acoustic cell filter, a hydrodynamic cell filter, a dead-end cell filter, a cell-stiffness sorter and a cross-flow cell filter. Additionally or alternatively, the sorting unit may also comprise a viscoelastic filter, a density sorter, a field-flow fractionation filter, a dielectrophoretic filter, a deterministic lateral displacement filter, and/or a weir filter.

An inertia-based cell filter may for example be configured to deflect a flow (i.e. induce a change in a direction of flow) of a carrier fluid containing the sample objects for sorting the sample objects. The inertia-based cell filter may e.g. comprise a channel with one or more curved portions, e.g. a spiral-shaped channel. The size and/or the weight of the sample objects may determine the extent to which the sample objects can follow the change in the flow direction. For example, lighter and/or smaller sample objects may be able to follow the deflected flow, whereas heavier and/or larger sample objects may not or only partially be able to follow the deflected flow, e.g. as a result of a centrifugal force acting on the sample objects. The first and second outlets of the sorting unit may be arranged such that smaller and/or lighter sample objects end up in the first outlet, whereas larger and/or heavier sample objects end up in the second outlet. A sorting range or threshold of the inertia filter may be controlled by adjusting a flow velocity in the inertia-based cell filter. Similarly, a hydrodynamic cell filter may be configured to sort the sample objects by generating size- and/or weight-dependent hydrodynamic forces on the sample objects.

An acoustic cell filter may for example be configured to sort the sample objects by generating a standing acoustic wave, e.g. a standing ultrasound wave. The acoustic wave may exert size-dependent forces on the sample objects, which may be used to deflect the sample objects into either the first or second outlet of the sorting unit depending on their size.

A dead-end cell filter and a cross-flow cell filter may comprise a mechanical filter structure such as a grid, a mesh, a lattice structure or a plate comprising a plurality of holes or perforations. The mechanical filter structure may be configured to prevent sample objects with a size larger than a sorting threshold (e.g. the size of the holes) from passing through the mechanical filter structure. In a dead-end cell filter, a carrier fluid containing the sample objects may be forced through the mechanical filter structure (thereby sorting out sample objects larger than the sorting threshold, which cannot pass through the filter structure), whereas in a cross-flow cell filter the carrier fluid may flow parallel to a surface of the mechanical filter structure (thereby sorting out sample objects smaller than the sorting threshold, which can pass through the filter structure and thus leave the carrier fluid flow).

The second outlet of the sorting unit may comprise a plurality of sub-outlets (e.g. between two and ten sub-outlets), for example to further sort the sample objects directed towards the second outlet similar to the sorting into the first and second outlets described above. For example, the sorting unit may be configured to sort the sample objects directed towards the second outlet by size, weight and/or stiffness by directing sample objects having a smaller size, a smaller weight and a lower stiffness, respectively, towards a first sub-outlet of the second outlet and sample objects having a larger size, a larger weight and a higher stiffness, respectively, towards a second sub-outlet of the second outlet, e.g. similar as described above. Each of the sub-outlets of the second outlet may for example be associated with a respective sorting threshold (e.g. a size and/or weight threshold) and/or a respective sorting range (e.g. a size and/or weight range).

In some embodiments, the sorting unit may comprise a waste outlet, e.g. to sort out dead and/or non-viable cells. The sorting unit may for example be configured to sort the sample objects by stiffness by directing sample objects having a higher stiffness from the inlet towards the waste outlet and sample objects having a lower stiffness from the inlet towards the first outlet and/or towards the second outlet (e.g. as a second sorting stage after sorting by size and/or weight). The waste outlet may for example be in fluid communication with a waste reservoir or container.

In some embodiments, the sorting unit may be configured to selectively direct sample objects into one of the dissociation volumes based on their size, weight and/or stiffness. For example, each of the sub-outlets of the second outlet may be associated with a respective means for dissociating sample objects and/or a respective dissociation volume, e.g. for dissociating sample objects by different means based on their size, weight and/or stiffness. For example, the first sub-outlet may be in fluid communication with a first dissociation volume, but not with a second dissociation volume, whereas the second sub-outlet may be in fluid communication with the second dissociation volume, but not with the first dissociation volume.

Similarly, the first outlet of the sorting unit may also comprise a plurality of sub-outlets to further sort the sample objects directed towards the first outlet by size, weight and/or stiffness. Each of the sub-outlets of the first outlet may for example be in fluid communication with a different measurement volume, e.g. a first sub-outlet with a first measurement channel for smaller sample objects and a second sub-outlet with a second measurement channel for larger sample objects as described below.

In a preferred embodiment, the microfluidic device is configured to sort sample objects dissociated in the dissociation unit again prior to transfer to the measurement volume, e.g. to prevent larger sample objects from entering the measurement volume and/or to perform additional dissociation steps for dissociating larger sample objects further. For this, the outlet of the dissociation unit may for example be in fluid communication with the measurement volume via the sorting unit. In other words, the dissociated sample objects may have to pass through the sorting unit again with only sample objects directed towards the first outlet thereof being transferred to the measurement chamber. For example, a channel from the outlet of the dissociation volume may intersect with a channel connecting the insertion volume to the sorting unit upstream of the sorting unit.

Additionally or alternatively, the outlet of the dissociation unit may further comprise a sorter (e.g. an additional sorting unit), which may be similar or identical to the sorting unit described above. The sorter may have a first outlet that is in fluid communication with the measurement volume. The sorter may further have a second outlet that is in fluid communication with the inlet of the dissociation unit. The sorter may be configured to sort the sample objects by size, weight and/or stiffness by directing sample objects having a smaller size, a smaller weight and a lower stiffness, respectively, towards the first outlet and sample objects having a larger size, a larger weight and a higher stiffness, respectively, towards the second outlet, e.g. as described above. In one example, the second outlet of the sorter comprises a plurality of sub-outlets, e.g. as described above for the sorting unit. In some embodiments, the second outlet of the sorter may not be in fluid communication with the measurement volume, but may for example be connected to a waste outlet or a waste reservoir of the microfluidic device, e.g. for discarding sample objects that have not been dissociated sufficiently.

In some embodiments, the microfluidic device further comprises a filter arranged between the outlet of the dissociation unit and the inlet of the measurement volume. Additionally or alternatively, said filter (or an additional filter similar to said filter) may be arranged between the first outlet of the sorting unit and the inlet of the measurement volume. The filter may for example be arranged at the inlet of the measurement volume or along a channel connecting the outlet of the dissociation unit to the inlet of the measurement volume, preferably along a common channel connecting the inlet of the measurement volume to the first outlet of the sorting unit and to the outlet of the dissociation unit. The filter may be configured to prevent sample objects having a size larger than a filtering threshold from entering the measurement volume. The filter may for example be or comprise a dead-end cell filter or a cross-flow cell filter as described above. Additionally or alternatively, the filter may be configured to filter out dead or non-viable cells. The filter may for example be or comprise a cell-stiffness sorter that is configured to filter or sort sample objects by stiffness, e.g. as described above for the sorting unit. This may for example allow to filter out dead or non-viable cells, which may exhibit a higher stiffness than living or viable cells, for example as described in M. Islam et al., Scientific Reports 7, 1997 (2017). In some embodiments, the filter may also be configured to exchange a fluid or medium (or a part thereof) that the sample objects are contained or suspended in, for example to exchange a carrier fluid and/or a dissolving solution by a different fluid or medium such as a measurement fluid. In one example, said filter or parts thereof may be comprised in the sorting unit.

The microfluidic device may comprise a sample collection unit for inserting a sample into the insertion volume. The sample collection unit may comprise one or more means for inserting a sample into the insertion volume, each of which may be configured to interface with a sample collection device. The sample collection unit may for example comprise a connector configured to couple a syringe and/or a tube containing a sample (e.g. a sample fluid) comprising the one or more sample objects to the insertion volume. Additionally or alternatively, the sample collection unit may comprise an opening configured to receive an aspiration needle, e.g. a fine-needle aspiration (FNA) needle, and/or a biopsy needle, e.g. a fine-needle biopsy (FNB) needle, wherein the opening is in fluid communication with the insertion volume.

Additionally or alternatively, the sample collection unit may comprise a transfer pump configured to transfer a sample from a collection tube (e.g. a standard centrifuge tube) to the insertion volume. The transfer pump may for example be configured to suck the sample out of the collection tube and to release the sample into the insertion volume. The transfer pump may also be configured to flush the collection tube, e.g. to detach a sample from a sample collection device such as a biopsy needle, a brush or a spatula. The collection tube may be part of the microfluidic device or may be a separate element. The collection tube may be removable or may be permanently integrated into the microfluidic device. In some examples, the transfer pump may be arranged in a moveable head. The moveable head may be configured to be inserted into the collection tube, e.g. for sucking a sample out of the collection tube using the transfer pump. The moveable head may further be configured to move the sample to the insertion volume, where the sample may be released, e.g. flushed out of the moveable head by the transfer pump.

The microfluidic device may further comprise a wiper for wiping off a sample from a sample collection device, in particular for wiping off a biopsy sample from a biopsy needle. A wiper may for example be arranged on a side wall of the insertion volume and/or on a side wall of the collection tube. The wiper may e.g. be a protrusion or a step that the sample can be brought in contact with for detaching the sample from the sample collection device.

The microfluidic device may further comprise a flow pump configured to generate a flow through the insertion volume to transport the sample objects from the insertion volume to the sorting unit. The flow pump may also be configured to flush and/or suck out the insertion volume, e.g. to detach a sample from a sample collection device such as a brush or a spatula and/or from the wiper. The flow pump may in particular be configured to detach a biopsy sample from a biopsy needle and/or from the wiper by flushing and/or sucking out the insertion volume. In some embodiments, the flow pump may also be the transfer pump.

In some embodiments, the measurement volume comprises a first measurement channel and a second measurement channel connected in parallel to the first measurement channel. The second measurement channel may have a larger cross-sectional area than the first measurement channel, e.g. for analyzing sample objects with a larger size. The cross-sectional area of the second measurement channel may for example be between 1.5 times and 5 times as large as the cross-sectional area of the first measurement channel. The microfluidic device may comprise a pump and/or a valve for selectively directing sample objects into either the first measurement channel or the second measurement channel. Additionally or alternatively, the sorting unit may be configured to selectively direct sample objects into either the first measurement channel (e.g. via a first sub-outlet of the first outlet of the sorting unit) or the second measurement channel (e.g. via a second sub-outlet of the first outlet of the sorting unit) based on a size, a weight and/or a stiffness of the respective sample object. The sorting unit may for example be configured such that sample objects having a smaller size, a smaller weight and/or a lower stiffness are directed into the first measurement channel and sample objects having a larger size, a larger weight and/or a higher are directed into the second measurement channel.

The microfluidic device may also comprise a measurement fluid inlet arranged upstream of the inlet of the measurement volume for supplying a measurement fluid. The measurement fluid inlet may in particular be arranged between the outlet of the dissociation unit and the inlet of the measurement unit. This may for example allow for replacing a carrier fluid used for transporting the sample objects through the sorting unit and/or through the dissociation unit, which may e.g. contain enzymes added in the dissociation unit, by a different fluid that measurements in the measurement volume are to be performed in, for example a viscoelastic fluid, and/or to add substances such as a viscoelastic substance to the carrier fluid.

In a preferred embodiment, the measurement volume comprises an optical detection window for taking microscopic images, in particular phase shift images of sample objects in the measurement volume. The detection window may have optical properties that allow for microscopic imaging and in particular phase shift imaging therethrough. The detection window may for example have a transmitted wavefront error of no more than λ/2, in some examples of no more than λ/4, in one example of no more than λ/8, wherein the transmitted wavefront error may e.g. be measured according to the ISO 10110 standard. In some embodiments, the detection window may have an anti-reflective coating.

The present invention further provides a method of analyzing a sample comprising one or more sample objects containing biological cells using a microfluidic device according to any one of the embodiments described therein. The one or more sample objects comprise one or more single cells, one or more cell aggregates and/or one or more pieces of continuous tissue material. The method comprises generating a flow of a carrier fluid through the insertion volume to transport sample objects from the insertion volume to the sorting unit. The sample objects are sorted with the sorting unit into a first batch of sample objects and a first residual sample. The first residual sample comprises sample objects having a larger size, a larger weight and/or a higher stiffness than the sample objects in the first batch. A first measurement is performed on the sample objects of the first batch in the measurement volume. Sample objects in the first residual sample are dissociated with the dissociation unit into single cells and/or cell aggregates at least in part to obtain a second batch of sample objects. A second measurement is performed on the dissociated sample objects in the second batch in the measurement volume.

The method may also comprise providing the sample in the insertion volume, e.g. via a connector or an opening as described above or by transferring the sample from a collection tube as described above. This may comprise wiping the sample off a sample collection device using a wiper and/or flushing and/or sucking out the insertion volume and/or the collection tube to detach the sample from a sample collection device. In other examples, the sample may be provided in the insertion volume prior to executing the method. A carrier fluid such as phosphate-buffered saline may be provided in the insertion volume. A flow of the carrier fluid may be generated from the insertion volume through the sorting unit to the measurement volume and to the dissociation unit to transport some or all of the sample objects in the insertion volume to the sorting unit and further on to either the measurement volume (first batch) or the dissociation unit (first residual sample).

The sample objects may be sorted as described above for the microfluidic device according to the invention with the sample object in the first batch being directed towards the first outlet of the sorting unit (and then to the measurement volume) and the sample objects in the first residual sample being directed towards the second outlet of the sorting unit (and then to the dissociation unit). The sample objects may for example be sorted such that the first batch contains sample objects having a size and/or a weight within a respective first range and/or below a respective sorting threshold, whereas the first residual sample contains sample objects having a size and/or a weight within a respective second range and/or above the respective sorting threshold with an upper bound of the first range being smaller than or equal to a lower bound of the second range. The first batch may for example contain single cells and cell aggregates, but no pieces of tissue or may contain single cells and small cell aggregates, but no large cell aggregates and no pieces of tissue. Additionally or alternatively, the sample objects may also be sorted by stiffness, e.g. as described above.

The sample objects may be dissociated into smaller objects as described above using one or more means for dissociating sample objects, e.g. by chemical dissociation and in particular enzymatic digestion. For example, a dissolving solution such as an enzymatic solution comprising one or more enzymes for dissolving sample objects by enzymatic digestion may be supplied to a dissociation volume that the sample objects are arranged in or flowing through. The sample objects may be exposed to the dissolving solution for a predetermined amount of time, e.g. between 1 min and 60 min, in one example between 5 min and 30 min. Additionally or alternatively, the sample objects may be dissociated mechanically, for example by applying acoustic waves (e.g. ultrasound), by generating a shear force and/or by mechanically dissociating the sample objects by static obstacles and/or moveable elements such as a stir bar or a grinder. In some embodiments, the sample objects may be dissociated sequentially using different means for dissociating sample objects, for example by first dissociating the sample objects by a first means (e.g. cutting and/or grinding sample objects mechanically) and then further dissociating the sample objects by a second means (e.g. dissolving the sample objects by enzymatic digestion).

The second batch of sample objects may be obtained by sorting the dissociated sample objects into the second batch of sample objects and a second residual sample, e.g. similar to the sorting of the sample objects into the first batch and the first residual sample with the sorting unit described above. The second residual sample may comprise sample objects having a larger size, a larger weight and/or a higher stiffness than the sample objects in the second batch. The dissociated sample objects may for example be sorted using the sorting unit or using a sorter at an outlet of the dissociation unit. The second batch may then be transferred to the measurement volume for performing the second measurement.

The second residual sample may either be discarded or may be dissociated further to obtain one or more additional batches of sample objects, e.g. by performing one or more additional dissociation and/or sorting steps. An additional measurement may be performed on dissociated sample objects in each of the additional batches in the measurement volume. The one or more additional batches of sample objects may for example be obtained by dissociating sample objects in a residual sample separated from a previous batch (e.g. the second residual sample separated from the second batch) into single cells and/or cell aggregates at least in part using the dissociation unit and sorting the dissociated sample objects into the respective additional batch of sample objects (e.g. the third batch) and a new residual sample (e.g. the third residual sample). The new residual sample may comprise sample objects having a larger size, a larger weight and/or a higher stiffness than the sample objects in the respective additional batch, e.g. as described above. The method may for example comprise obtaining between 1 and 20 batches, in one example between 3 and 10 batches of sample objects from the sample. In some embodiments, different means for dissociating sample objects may be used for different dissociation steps. For example, mechanical means may be used in a first dissociation step while chemical dissociation (e.g. enzymatic digestion), ultrasound and/or shear force may be used in one or more later dissociation steps.

In this way, the sample objects may be dissociated sequentially and separated into a plurality of batches. Each of the batches may for example only contain objects within a certain size range, e.g. only objects smaller than a sorting threshold. This may allow for performing measurements on the objects in each of the batches on the single cell level and may further prevent larger objects from clogging the measurement volume. Each of the batches may be associated with certain types of sample objects and/or certain parts of the sample objects and may thus provide unique information on the original sample and its composition. For example, the first batch may predominantly contain single cells and/or small cell aggregates that were already contained in the original sample and the second batch may predominantly contain single cells and/or small cell aggregates originating from larger cell aggregates and/or from a surface layer of pieces of tissue from the original sample. Subsequent batches may predominantly contain single cells and/or small cell aggregates originating from deeper and deeper layers of pieces of tissue from the original sample, thereby allowing for a spatially resolved analysis of the pieces of tissue.

In some embodiments, the method may comprise filtering some or all of the batches of sample objects before the respective batch enters the measurement volume, e.g. to prevent sample objects having a size larger than a filtering threshold from entering the measurement volume and/or to remove dead and/or non-viable cells. The batches may for example be filtered using a filter arranged upstream of the measurement volume as detailed above for the microfluidic device according to the invention.

The method may comprise determining a sample type of the sample, wherein the sample type characterizes one or more of a procedure used for obtaining the sample from a patient (e.g. biopsy, aspiration, resection, puncture, lavage or smear), a body part that the sample was obtained from (e.g. an organ, a type of tissue or a type of body fluid), a size of the sample objects in the sample (e.g. an average size and/or a maximum size) and a weight of the sample objects in the sample. The sample type may for example be determined based on a user input (the user may e.g. enter or specify the sample type using an input device such as a touchscreen or touch-pad) and/or based on a label such as a barcode associated with the sample and/or a sample collection device. The method may further comprise determining a number of dissociation steps that are to be performed based on the sample type and performing the dissociation steps and sorting the dissociated sample objects by size, weight and/or stiffness to obtain a plurality of batches of sample objects. For example, the larger the size of the sample objects in the sample is the more dissociation steps may be performed.

The sample may for example comprises one or more of a biopsy sample (e.g. a fine-needle biopsy sample), an aspiration sample (e.g. a fine-needle aspiration sample), a body fluid sample (wherein the body fluid may e.g. be blood, lymph, an effusion (e.g. a pleural, peritoneal or pericardial effusion), cerebrospinal fluid, urine or combinations thereof), a smear sample (e.g. an oral smear, a cervical smear and/or a bone smear) and/or a resection sample (e.g. tissue pieces from an excision and/or resection such as sonicated brain tissue of resection rim).

A smaller number or no dissociation steps may be performed if the sample type indicates that the sample is a body fluid sample and/or if the sample type indicates that the sample is a smear sample, while a larger number of dissociation steps may be performed if the sample type indicates that the sample is a biopsy sample, if the sample type indicates that the sample is an aspiration sample and/or if the sample type indicates that the sample is a resection sample. In one example, no dissociation step or a single dissociation step is performed if the sample type indicates that the sample is a body fluid sample and/or if the sample type indicates that the sample is a smear sample and multiple dissociation steps (e.g. between two and ten dissociation steps) are performed if the sample type indicates that the sample is a biopsy sample and/or if the sample type indicates that the sample is an aspiration sample. Similarly, a larger number of dissociation steps may be performed if the sample type indicates that the sample is a biopsy sample than if the sample type indicates that the sample is an aspiration sample.

In some embodiments, the dissociation unit of the microfluidic device comprises a plurality of means for dissociating the sample objects. The method may further comprise selecting a means for dissociating the sample objects from the plurality of means for dissociating the sample objects based on the sample type. For example, if the sample type indicates that the sample is a body fluid sample and/or if the sample type indicates that the sample is a body fluid sample, the sample objects may be dissociated by chemical dissociation (e.g. enzymatic digestion), whereas if the sample type indicates that the sample is a biopsy sample, if the sample type indicates that the sample is a resection sample and/or if the sample type indicates that the sample is an aspiration sample the sample objects may be dissociated by mechanical means, e.g. in addition to chemical dissociation/enzymatic digestion.

Preferably, a plurality of dissociation protocols are provided, e.g. on a storage medium such as a storage medium of the controller of the system according to the invention described below. One of said dissociation protocols may be selected based on the sample type. Each dissociation protocol may specify a number of dissociation steps that are to be performed and/or means for dissociation sample objects that are to be used. The plurality of dissociation protocols may for example be provided as a library, in particular as an extendable library, to which a user may e.g. add further dissociation protocols.

In some embodiments, the method may comprise performing one or more control measurements on some or all of the batches of sample objects prior to performing the (main) measurement on the sample objects of the respective batch in the measurement volume. The one or more control measurements may for example comprise determining an object count and/or a cell count (e.g. a total number or density of sample objects and cells, respectively, in the respective batch) and/or a cell viability measure (e.g. a percentage of viable cells in the respective batch) for one or more of the batches of sample objects. The control measurements may for example be performed by optical means (e.g. using an optical sensor such as a photodiode or a camera), by electrical means (e.g. using one or more electrodes to determine a conductivity, an impedance and/or a permittivity) and/or by mechanical means (e.g. for sorting cells by one or more characteristics such as viability).

The method may comprise manipulating the batches based on a result of the control measurements prior to performing the main measurements in the measurement volume. In particular, a density of sample objects of the respective batch may be adjusted based on the determined object count by supplying a carrier or measurement fluid, e.g. through the measurement fluid inlet of the microfluidic device. For example, fluid may be added if the object density of a batch is above a certain threshold or a variable amount of fluid may be added depending on the object density. This may ensure that the density of sample objects in the batch is sufficiently small such that different sample objects can be analyzed separately.

The first measurement and the second measurement as well as any additional measurement on sample objects in the measurement volume are not particularly limited to any detection or measurement technique, but may be any type of measurement suitable for analyzing single cells and/or cell aggregates. The measurements may for example be or may comprise an electrical measurement (e.g. a measurement for determining a conductivity, an impedance and/or a permittivity) and/or an optical measurement (e.g. a measurement for determining an intensity of light transmitted, scattered, reflected and/or emitted by the sample objects).

Preferably, the measurements correspond to or comprise taking one or more microscopic images, in particular phase shift images of sample objects in the measurement volume. In other words, performing the first measurement and/or performing the second measurement may comprise or correspond to taking an image of the sample objects in the measurement volume using a microscope, in particular taking a phase shift image of the sample objects in the measurement volume using a phase-contrast microscope, in particular a quantitative phase-contrast microscope, for example a digital holographic microscope. As used herein, a phase shift image may refer to an image that encodes a phase shift of light (e.g. an absolute phase shift or a phase shift modulo 2π) at one or more wavelengths as a function of position. The phase shift may for example be the phase shift of light that is transmitted through or reflected off the sample objects in the measurement volume.

Additionally or alternatively, different microscopy techniques such as bright-field microscopy, fluorescence microscopy, and/or super-resolution microscopy may be used, e.g. to obtain an amplitude or intensity image. In some examples, the measurements may also be or comprise one or more spectroscopic measurements on the sample objects in the measurement volume, wherein a spectroscopic measurement may for example comprise recording one or more optical spectra of one or more sample objects, e.g. an absorption spectrum and/or an emission spectrum in one or more wavelength ranges.

Performing the first measurement, the second measurement and/or any additional measurement may comprise hydrodynamically and/or viscoelastically focusing the respective sample objects in the measurement volume, for example in a focal plane of a microscope. Hydrodynamic focusing may comprise generating one or more sheath flows, in particular laminar sheath flows, surrounding a carrier or measurement fluid flow in the measurement volume, e.g. to confine sample objects in the carrier or measurement fluid flow along one or more directions to a smaller volume, for example at the center of the measurement volume or in the vicinity of a sidewall of the measurement volume. Viscoelastic focusing may for example be achieved by providing a viscoelastic carrier or measurement fluid, e.g. a fluid containing a shear-thinning polymer such as poly(ethylene oxide) (PEO) and/or poly(vinyl pyrrolidone) (PVP). A viscoelastic fluid may exert hydrodynamic forces on the sample objects, thereby inducing a controlled motion perpendicular to a direction of flow.

In some embodiments, the method may also comprise performing an analysis of sample objects based on the first measurement, the second measurement and/or any additional measurements. This may for example comprise identifying sample objects and/or their constituents (e.g. individual cells) in a microscopic image, e.g. using classical and/or artificial intelligence-based (AI-based) computer vision techniques such as image segmentation algorithms and/or neural network-based classifiers. This may also comprise determining one or more parameters, in particular morphological parameters, of sample objects and/or their constituents in a microscopic image such as a size (e.g. an equivalent diameter), an optical height (e.g. a mean phase shift and/or a maximum phase shift), an aspect ratio, a number of cells in the respective sample object and/or a type of the cells in the respective sample object. This may for example allow for obtaining a distribution of these parameters over the sample objects and/or cells of a batch as well as for performing single-cell phenotyping of the cells contained in the sample objects. The results of the analysis of the sample objects may be used for diagnostic, prognostic and/or therapeutic purposes, for example to diagnose a medical condition, to assess a severity of a medical condition such as the malignancy of a cancer and/or to select a certain therapy such as a certain drug. In some embodiments, the measurements may be used to probe a stiffness and/or an elasticity of the sample objects, e.g. as described in D. R. Gossett, Proc. Natl. Acad. Sci. 109(20), 7630-7635 (2012). A higher stiffness may indicate malignancy in some examples.

The invention further provides a system for analyzing a sample comprising one or more sample objects containing biological cells, the one or more sample objects comprising one or more single cells, one or more cell aggregates and/or one or more pieces of continuous tissue material. The system is configured for use with a microfluidic device according to any one of the embodiments described herein. The system comprises a microfluidics unit configured to generate a flow of a carrier fluid through the insertion volume of the microfluidic device to transport sample objects from the insertion volume to the sorting unit of the microfluidic device. The system further comprises a measurement device configured to perform measurements on single cells and/or cell aggregates in the measurement volume of the microfluidic device. The system also comprises a controller configured to control the microfluidics unit, the measurement device, the sorting unit of the microfluidic device and/or the dissociation unit of the microfluidic device to execute a method according to any one of the embodiments described herein.

The system may be configured to receive a microfluidic device according to any one of the embodiments described herein. The system may for example comprise a mount that is configured to receive the microfluidic device. The mount may further be configured to provide fluid, electrical and/or optical connections to the microfluidic device, e.g. to connect the microfluidic device to the microfluidics unit and/or to the controller. In some embodiments, the microfluidic device or parts thereof may be integrated into the system.

The microfluidics unit may comprise one or more pumps and/or one or more valves for generating and controlling fluid flows within the microfluidic device. Additionally or alternatively, the microfluidics unit may be configured to control one or more pumps and/or one or more valves integrated into the microfluidic device for generating and controlling fluid flows within the microfluidic device, e.g. via corresponding electrical and/or fluid connections. The microfluidics unit may further comprise one or more reservoirs for storing fluids such as the carrier fluid, one or more cleaning fluids, one or more dissolving or enzymatic solutions and/or a measurement fluid and for supplying said fluids to the microfluidic device. The microfluidics unit may for example comprise a reservoir storing a cleaning fluid, wherein the cleaning fluid may e.g. comprise a chemical substance such as hypochloride and/or a cleaning enzyme for cleaning the microfluidic device or a part thereof. The microfluidics unit may be configured to provide the cleaning solution to the microfluidic device or said part thereof, e.g. to flush the microfluidic device or said part thereof with the cleaning solution. In some embodiments, the reservoir for storing the cleaning fluid may also be comprised in the microfluidic device itself.

The measurement device may for example be configured to perform electrical and/or optical measurements on single cells and/or cell aggregates in the measurement volume. The measurement device may for example be or comprise a microscope configured to take microscopic images of single cells and/or cell aggregates in the measurement volume.

In a preferred embodiment, the measurement device is or comprises a phase-contrast microscope, in particular a quantitative phase-contrast microscope such as a digital holographic microscope configured to take phase shift images of single cells and/or cell aggregates in the measurement volume of the microfluidic device. The microscope may for example be configured to obtain the phase shift through interference of light, e.g. between a probe or imaging beam and a reference beam. In other examples, the quantitative phase-contrast microscope may be a ptychographic imaging device that is configured to perform ptychographic imaging without a reference beam, e.g. by recording interference patterns without a reference phase. The quantitative phase-contrast microscope may be configured to take one or more interference images and to reconstruct a phase shift image from the one or interference images.

Preferably, the quantitative phase-contrast microscope is a digital holographic microscope, which is configured to take quantitative phase shift images as well as amplitude or intensity images, wherein an intensity image may encode an intensity of light as a function of position, e.g. an intensity of light reflected off or transmitted through an imaging sample such as the sample objects in the measurement volume as a function of the position in the imaging sample. The digital holographic microscope may for example be configured to interfere an image of the imaging sample, e.g. an imaging beam of light transmitted through the imaging sample, with a reference beam, wherein the reference beam may pass through the imaging sample or may not pass through the imaging sample. The digital holographic microscope may be configured to extract or reconstruct the phase shift and intensity images from one or more interference images, e.g. by reconstructing a wave front of light transmitted through or reflected off the imaging sample. The digital holographic microscope may be an on-axis digital holographic microscope, in which the imaging beam and the reference beam propagate along the same axis when interfering. Preferably, the digital holographic microscope is an off-axis digital holographic microscope, in which the imaging beam and the reference beam interfere under an angle and which may be configured to extract a phase shift image from a single interference image of the imaging sample. Such digital holographic microscopes are for example known from EP 1 524 491 A1 and EP 2 357 539 A1.

The controller may be implemented in hardware, software or a combination thereof. The controller may for example comprise a processing device and a storage medium, wherein the storage medium contains instructions for execution by the processing device to provide the functionality described herein. The storage medium may further be configured to store data pertaining to the measurements performed by the measurement device (e.g. images and/or data extracted from the images). In some embodiments, the controller and/or the system may additionally or alternatively comprise a second storage medium for storing said data. The system may further comprise a communication module for transferring said data to an external device, for example via a network connection and/or via the internet.

The system may also comprise one or more means for dissociating sample objects into single cells and/or cell aggregates at least in part. The system may for example comprise an acoustic source, in particular an ultrasound source, that is configured to apply acoustic waves (e.g. ultrasound) to sample objects in the dissociation unit of the measurement device. The controller may be configured to control the acoustic source to dissociate sample objects in the dissociation unit by applying acoustic waves to the sample objects.

In some embodiments, the microfluidics unit may be configured to provide a dissolving solution (e.g. an enzymatic solution) to the dissociation unit of the microfluidic device, the dissolving solution comprising one or more chemical and/or biochemical substances for chemically dissolving sample objects, for example by enzymatic digestion, e.g. as detailed above. The controller may be configured to control the microfluidics unit to dissociate sample objects in the dissociation unit by supplying the dissolving solution to the dissociation unit.

In some embodiments, the system may comprise a stirrer, particularly a magnetic stirrer, that is configured to move a stir bar, particularly a magnetic stir bar, in the dissociation unit of the microfluidic device, e.g. as described above. The magnetic stirrer may for example be arranged on or in the mount for the microfluidic device.

In some examples, the device may also comprise one or more microfluidic devices according to any one of the embodiments described herein. The microfluidic device may be a removable component of the system according to the invention (e.g. such that the microfluidic device can be exchanged against a different microfluidic device) or may be permanently integrated into the system. Additionally or alternatively, one or more of the components of the microfluidic device according the invention described herein may be part of the system according to the invention instead of being integrated into the microfluidic device. For example, the dissociation unit (or a part thereof) may not be part of the microfluidic device, but may be comprised in the system instead, wherein the dissociation unit may e.g. be configured to be connected to the microfluidic device via a corresponding inlet and outlet of the microfluidic device. Some or all of the components of the microfluidic device that are integrated into the system may be replaceable, e.g. at least the insertion volume, the sorting unit and/or the dissociation unit, and may for example be embodied as modules that can be replaced individually, e.g. as described above for the microfluidic device according to the invention.

### LIST OF FIGURES

In the following, a detailed description of the invention and exemplary embodiments thereof is given with reference to the figures. The figures show schematic illustrations of
Fig. 1: a microfluidic device for analyzing a sample comprising one or more sample objects containing biological cells according to an exemplary embodiment of the invention;
Fig. 2a: a microfluidic device comprising a plurality of dissociation volumes in accordance with an exemplary embodiment of the invention;
Fig. 2b: a microfluidic device comprising a plurality of dissociation volumes in accordance with another exemplary embodiment of the invention;
Fig. 3: a microfluidic device comprising a sample collection unit and a preparatory unit according to an exemplary embodiment of the invention;
Fig. 4: sample collection units for a microfluidic device in accordance with exemplary embodiments of the invention;
Fig. 5: the insertion of samples into the sample collection units of Fig. 4 according to exemplary embodiments of the invention;
Fig. 6: a system for analyzing a sample comprising one or more sample objects containing biological cells according to an exemplary embodiment of the invention;
Fig. 7: a flow chart of a method of analyzing a sample comprising one or more sample objects containing biological cells according to an exemplary embodiment of the invention; and
Fig. 8: experimental data demonstrating the sorting and dissociation of sample objects containing biological cells.

### DESCRIPTION OF THE PREFERRED EMBODIMENTS

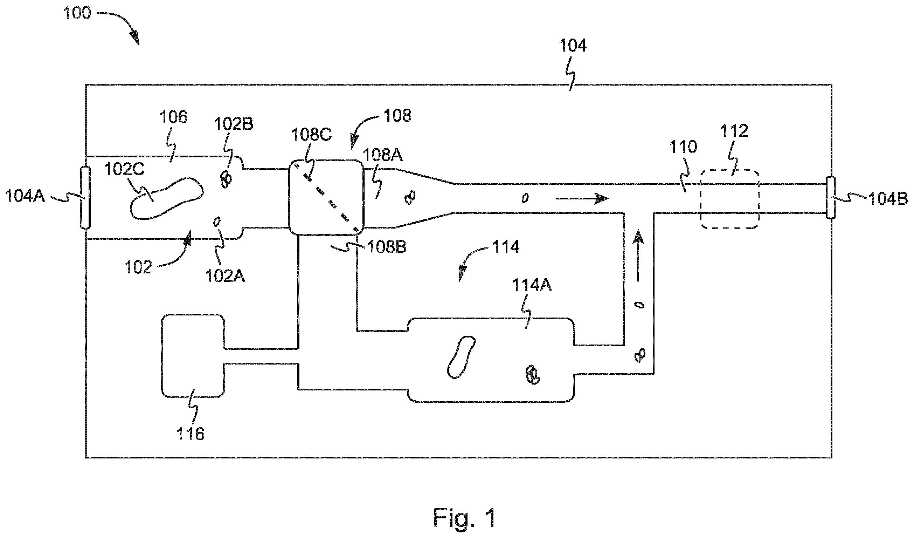

Fig. 1 shows a schematic illustration (not to scale) of a microfluidic device 100 for analyzing a sample 102 comprising sample objects 102A-C containing biological cells according to an exemplary embodiment of the invention. The sample 102 comprises a plurality of single cells 102A, a plurality of cell aggregates 102B as well as one or more pieces of continuous tissue material 102C. The sample 102 may for example be a biopsy or aspiration sample obtained from a patient, e.g. from cancer tissue in an organ of the patient. In other examples, the sample 102 may for example be a body fluid sample such as a blood sample or a smear sample such as an oral smear.

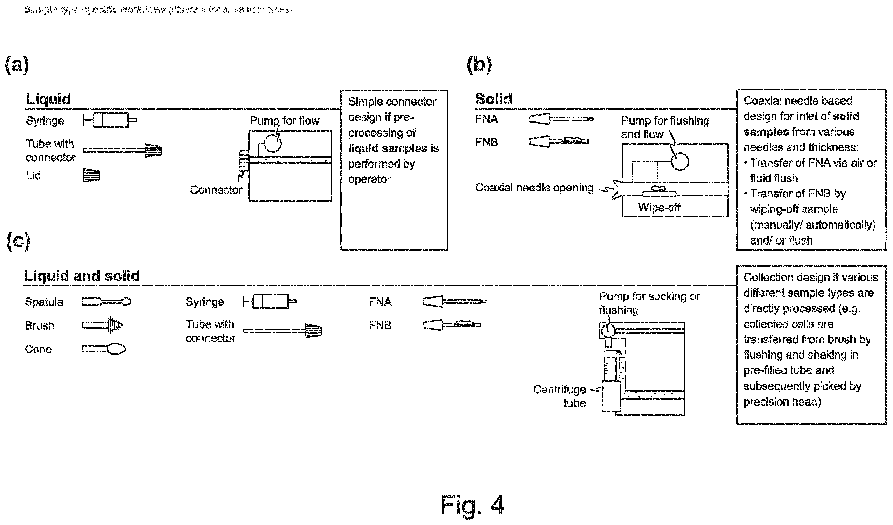

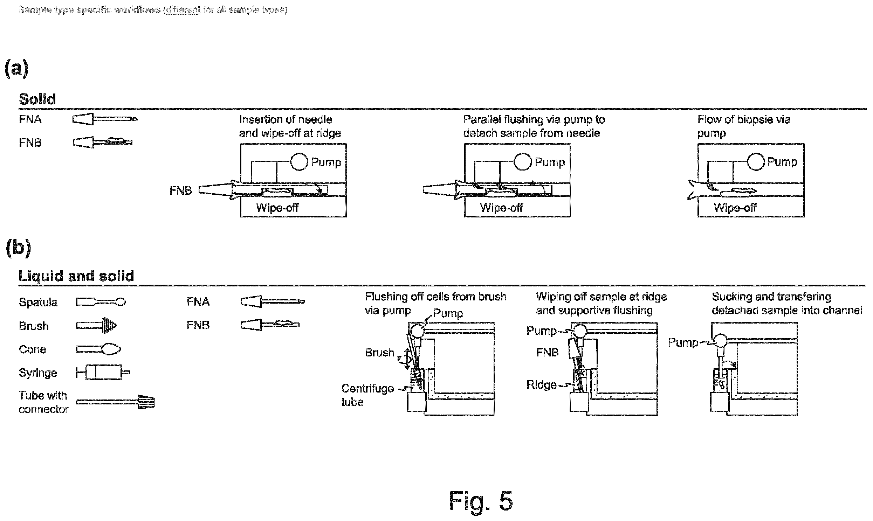

The microfluidic device 100 comprises a substrate 104, which may for example comprise or consist of a transparent thermoplastic such as polymethyl methacrylate (PMMA). An insertion volume 106 is formed in the substrate 104, wherein the insertion volume 106 is configured to receive the sample 102, e.g. through an inlet or opening 104A. A width, a height and a length of the insertion volume 106 may for example be between 0.5 mm and 50 mm, in one example between 1 mm and 10 mm. A volume of the insertion volume may for example be between 1 mm³ and 20 cm³. The insertion volume 106 may be part of a sample collection unit as detailed below with reference to Figs. 4 and 5.

The microfluidic device 100 further comprises a sorting unit 108 that is configured to sort the sample objects 102A-C by size, weight and/or stiffness. An inlet of the sorting unit 108 is in fluid communication with the insertion volume 106 via a microfluidic channel. A first outlet 108A of the sorting unit 108 is in fluid communication with a measurement volume 110 formed in the substrate 104 via another microfluidic channel. A second outlet 108B of the sorting unit 108 is in fluid communication with a dissociation unit 114 via yet another microfluidic channel.

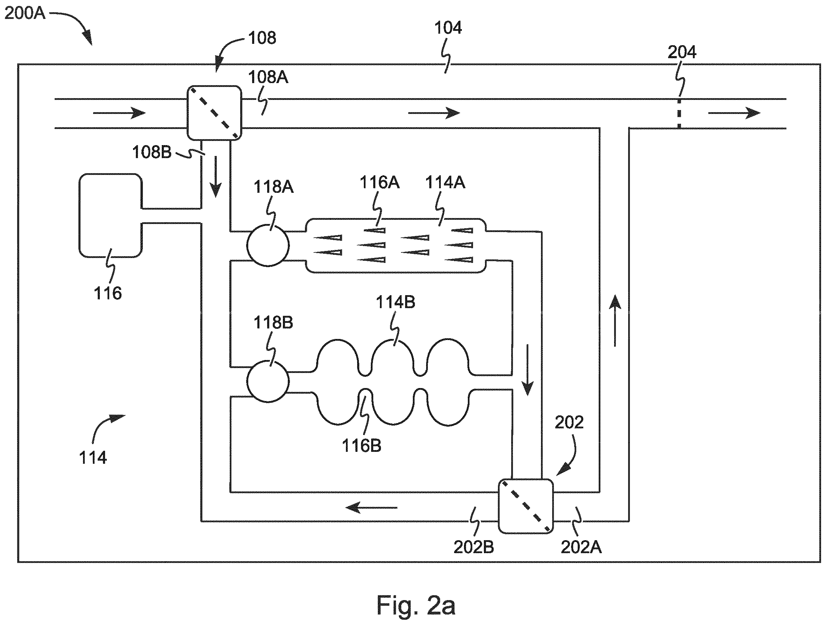

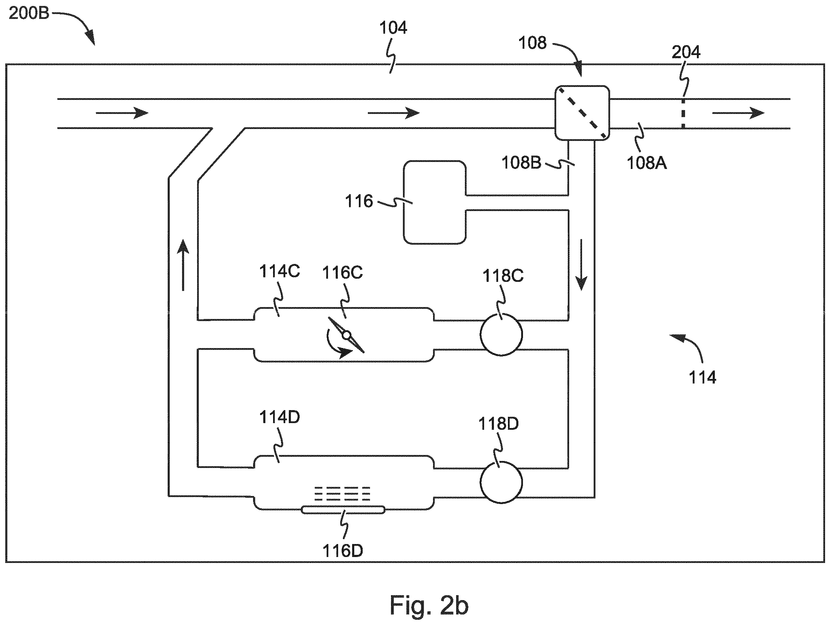

The sorting unit 108 comprises a filter 108C, which may for example be an inertia-based cell filter, an acoustic cell filter, a hydrodynamic cell filter, a dead-end cell filter or a cross-flow cell filter. In some embodiments, the sorting unit 108 may comprise a plurality of filters selected from the aforementioned types of filters, which may for example be arranged in series. The filter 108C is configured to direct sample objects having a smaller size, a smaller weight and/or a lower stiffness from the inlet towards the first outlet 108A. The filter 108C is further configured to direct sample objects having a larger size, a larger weight and/or a higher stiffness from the inlet towards the second outlet 108B. The filter 108C may for example be configured to direct sample objects having a size smaller than a (first) sorting threshold towards the first outlet 108A and sample objects having a size larger than the sorting threshold towards the second outlet 108B. The sorting threshold may for example be between 5 µm and 1000 µm, in some examples between 10 µm and 100 µm, e.g. 40 µm. This may for example allow for separating single cells 102A and smaller cell aggregates 102B from larger cell aggregates 102B and pieces of tissue 102C. The single cells 102A and the smaller cell aggregates 102B may pass through the filter 108C to the measurement volume 110, while the larger cell aggregates 102B and pieces of tissue 102C may be diverted to the dissociation unit 114. The sorting threshold may for example be chosen based on the physical dimensions of the measurement volume 110 (e.g. to prevent clogging thereof) and/or based on hydrodynamic considerations, for example to facilitate a hydrodynamic and/or viscoelastic focusing of sample objects in the measurement volume 110. In some embodiments, the microfluidic device 100 may further comprise another filter (not shown) and/or another sorter (not shown) between the outlet of the dissociation unit 114 and the inlet of the measurement volume 110, e.g. as described below with reference to Figs. 2a, 2b.

The measurement volume 110 is configured for performing measurements on sample objects contained therein, for example electrical and/or optical measurements. In one example, the measurement volume 110 is a microfluidic channel, which may for example have a width between 10 µm and 1000 µm, in one example between 30 µm and 500 µm, and a height between 10 µm and 1000 µm, in some examples between 30 µm and 500 µm, in one example between 30 µm and 100 µm. The measurement volume 110 may for example comprise an optical detection window 112, wherein the detection window 112 is configured for performing microscopic imaging and in particular phase-contrast imaging therethrough. A transmitted wavefront error of the detection window 112 may for example be no more than λ/2. Additionally or alternatively, the microfluidic device 100 may for example comprise one or more electrodes for performing electrical measurements in the measurement volume 110. The measurement volume 110 may be in fluid communication with an outlet or opening 104B and/or with a waste reservoir (not shown) of the microfluidic device 100.

The dissociation unit 114 comprises a dissociation volume 114A, wherein an inlet of the dissociation volume 114A is in fluid communication with the second outlet 108B of the sorting unit 108 and an outlet of the dissociation volume 114A is in fluid communication with the measurement volume 110 as illustrated in Fig. 1. The dissociation unit 114 comprises means for dissociating sample objects into smaller objects such as single cells and/or cell aggregates at least in part. In the example of Fig. 1, the means for dissociation sample objects comprise a reservoir 116 that is configured to store a dissolving solution, in particular an enzymatic solution, and to supply said solution to the dissociation volume 114A. The enzymatic solution may comprise one or more enzymes for dissolving sample objects by enzymatic digestion, for example trypsin and/or collagenase. The sample objects may be exposed to the enzymatic solution in the dissociation volume 114A and may thus be dissociated into smaller objects prior to being transferred to the measurement volume 110.

Fig. 2a shows a schematic illustration (not to scale) of a microfluidic device 200A according to another exemplary embodiment of the invention. The microfluidic device 200A is similar to the microfluidic device 100 of Fig. 1 and also comprises a sorting unit 108, a dissociation unit 114, an insertion volume (not shown) upstream of the sorting unit 108 and the dissociation unit 114 (i.e. to the left of the arrangement shown in Fig. 2) as well as a measurement volume (not shown) downstream of the sorting unit 108 and the dissociation unit 114 (i.e. to the right of the arrangement shown in Fig. 2).

In the example of Fig. 2a, the dissociation unit 114 comprises a plurality of dissociation volumes or dissociation channels, namely a first dissociation volume 114A and a second dissociation volume 114B, and a plurality of means for dissociating sample objects. The dissociation volumes 114A-B are arranged in parallel with each dissociation volume 114A-B being associated or equipped with a different means for dissociating sample objects. Furthermore, each dissociation volume 114A-B is associated or equipped with a means 118A-B for controlling a fluid flow through the respective dissociation volume 114A-B. Said means 118A-B may for example be or comprise a pump and/or a valve, e.g. such that sample objects may be selectively directed into one of the dissociation volumes 114A-B.

The means for dissociating sample objects comprise a reservoir 116 for supplying a dissolving solution, for example an enzymatic solution, which is arranged upstream of each of the dissociation volumes 114A-B, e.g. such that the dissolving/enzymatic solution can be supplied to each of the dissociation volumes 114A-B simultaneously. In some embodiments, the reservoir 116 may comprise a plurality of sub-reservoirs, each of which may for example be configured to supply a different dissolving or enzymatic solution, e.g. a solution containing a different substance/enzyme or a different combination of substances/enzymes.

The means for dissociating sample objects further comprise a grid or array of static obstacles 116A arranged in the first dissociation volume 114A, wherein the static obstacles 116A are configured to mechanically dissociate sample objects colliding with one of the obstacles 116A. Each of the static obstacles 116A may for example be or may comprise a blade that is configured to cut or slice through a sample object upon impact.

The means for dissociating sample objects further comprises a plurality of constricted or narrower portions 116B that are arranged along the second dissociation volume 114B in between a plurality of non-constricted or wider portions. Physical dimensions of the constricted portions 116B (e.g. a width in the drawing plane of Fig. 2a and/or a height perpendicular to the drawing plane of Fig. 2a) and/or a radius of curvature of the sidewalls of the second dissociation volume 114A are chosen such that strong shear forces are generated on sample objects flowing through the second dissociation volume 114B that are sufficiently large to break sample objects apart, e.g. to separate cells of a loosely-bound cell aggregate. In some embodiments, a cross-sectional area, a width and/or a height of the constricted portions may decrease successively along the second dissociation volume 114B (not shown).

The microfluidic device 200A comprises a sorter 202 that is arranged at the outlet of the dissociation unit 114. The sorter 202 may be similar or identical to the sorting unit 108 and is also configured to sort sample objects by size, weight and/or stiffness by directing sample objects having a smaller size(e.g., a size below a second sorting threshold, which may be equal to the first sorting threshold of the sorting unit 108), a smaller weight and/or a lower stiffness towards a first outlet 202A that is in fluid communication with the measurement volume and by directing sample objects having a larger size(e.g. a size above the second sorting threshold), a larger weight and/or a higher stiffness towards a second outlet 202B. The second outlet 202B is in fluid communication with an inlet of the dissociation unit 114, e.g. such that larger sample objects may cycle through the dissociation unit 114 repeatedly for sequentially dissociating larger sample objects. In other embodiments, the outlet of the dissociation unit 114 may be in fluid communication with the measurement volume via the sorting unit 108 (i.e. the sorting unit 108 may also provide the functionality of the sorter 202), e.g. by connecting the outlet of the dissociation unit 114 with an inlet upstream of the sorting unit 108, for example as detailed below with reference to Fig. 2b.

The microfluidic device 200A also comprises one or more filters 204 that are arranged upstream of the measurement volume between the first outlet 108A of the sorting unit 108 and the outlet of the dissociation unit 114, respectively, and the inlet of the measurement volume. The filters 204 may for example comprise one or more mechanical filters such as a dead-end cell filter and/or a cross-flow cell filter, e.g. to prevent sample objects larger than a filtering threshold, which may for example have passed through the sorting unit 108 or the sorter 202 accidentally, from entering the measurement volume. The filtering threshold may be equal to the first and/or second sorting thresholds. In other examples, the filtering threshold may be larger than the first and/or second sorting thresholds. In some embodiments, the filters 204 may comprise a cell-stiffness sorter that is configured to filter or sort sample objects by stiffness, e.g. to filter out dead and/or non-viable cells upstream of the measurement volume.

Fig. 2b shows a schematic illustration (not to scale) of a microfluidic device 200B according to yet another exemplary embodiment of the invention. The microfluidic device 200B is similar to the microfluidic device 200A of Fig. 2a and also comprises a sorting unit 108, a dissociation unit 114, an insertion volume (not shown) upstream of the sorting unit 108 and the dissociation unit 114 (i.e. to the left of the arrangement shown in Fig. 2) as well as a measurement volume (not shown) downstream of the sorting unit 108 and the dissociation unit 114 (i.e. to the right of the arrangement shown in Fig. 2).

Similar to the microfluidic device 200A, the dissociation unit 114 comprises a plurality of dissociation volumes or dissociation channels, namely a first dissociation volume 114C and a second dissociation volume 114D, and a plurality of means for dissociating sample objects. The means for dissociating sample objects comprise a reservoir 116 for supplying a dissolving solution, e.g. as detailed above for the microfluidic device 200A.

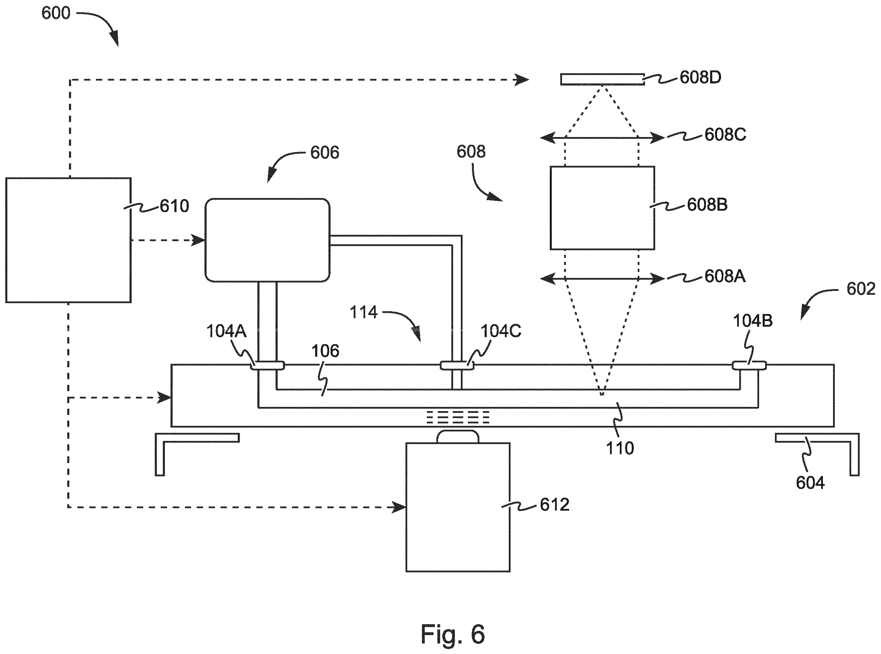

The means for dissociating sample objects further comprise a magnetic stirrer 116C that is configured to rotate a magnetic stir bar in the first dissociation volume 114C for dissociating sample objects. The magnetic stir bar may for example also comprise one or more blades configured to cut or slice through a sample object. The means for dissociating sample objects also comprise an acoustic emitter, in this case an ultrasound emitter 116D, arranged in a side wall of the second dissociation volume 114D, wherein the ultrasound emitter 116D is configured to apply ultrasound to sample objects in the second dissociation volume 114D. The ultrasound emitter 116D may for example be an active emitter comprising a piezo element, a capacitive element and/or an inductive element that is configured to convert an electrical signal applied to the ultrasound emitter to ultrasound. In other examples, the ultrasound emitter 116D may be a passive emitter, which may for example be configured to couple ultrasound generated by an external source such as a sonotrode into the second dissociation volume 114D, e.g. as described below for the device 600 of Fig. 6.

In the example of Fig. 2b, the microfluidic device 200B does not comprise an additional sorter such as the sorter 202 of the microfluidic device 200A. Instead, the outlet of the dissociation unit 114 is in fluid communication with the measurement volume via the sorting unit 108 (i.e. the sorting unit 108 also provides the functionality of the sorter 202). For this, the outlet of the dissociation unit 114 is connected to an inlet upstream of the sorting unit 108. Sample objects dissociated in the dissociation unit 114 thus have to pass through the sorting unit 108 again before entering the measurement volume.

In some embodiments, the microfluidic device 200A and/or the microfluidic device 200B may comprise more than two dissociation volumes and/or further means for dissociating sample objects, for example some or all of the dissociation volumes 114A-D and/or some or all of the means 116, 116A-D for dissociating sample objects as described above.

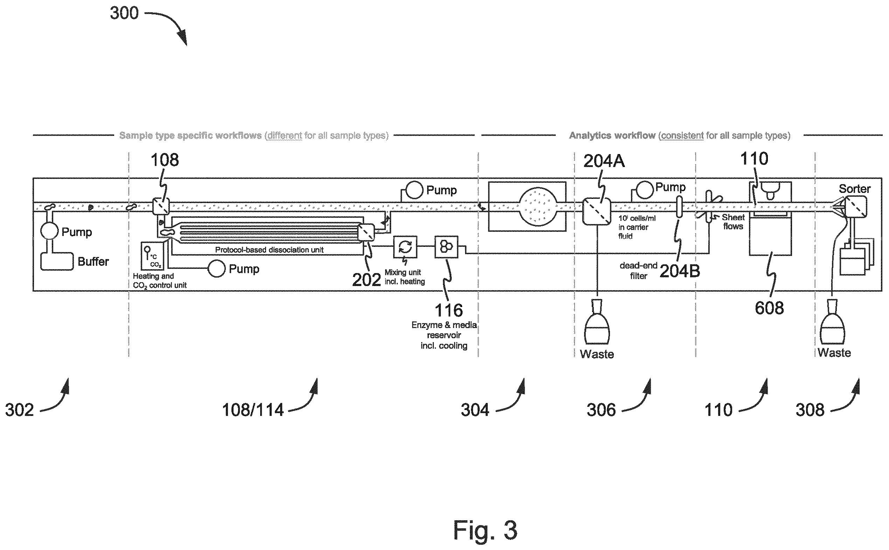

Fig. 3 shows a schematic illustration (not to scale) of a microfluidic device 300 according to yet another exemplary embodiment of the invention. The microfluidic device 300 is similar to the microfluidic devices 100, 200A, 200B of Figs. 1, 2a and 2b, respectively, and comprises a sample collection unit 302 with an insertion volume for receiving a sample, a dissociation and filtration unit with a sorting unit 108 and a dissociation unit 114 downstream of the sample collection unit 302 as well as a measurement volume 110 downstream of the dissociation and filtration unit. In the measurement volume 110, measurements may be performed with a measurement device 608 such as a digital holographic microscope, for example using the system 600 of Fig. 6 described below.

The sample collection unit 302 comprises a reservoir for supplying a buffer or carrier fluid such as phosphate-buffered saline (PBS) and a flow pump configured to generate a flow of the carrier fluid through the insertion volume to transport sample objects from the insertion volume to the sorting unit 108 and further on to the dissociation unit 114 and the measurement volume 110. The sample collection unit 302 may for example be embodied as detailed below with reference to Figs. 4 and 5.

The dissociation and filtration unit with the sorting unit 108 and the dissociation unit 114 may for example be embodied as in the microfluidic devices 200A, 200B of Figs. 2a, 2b. The dissociation and filtration unit 108/114 may comprise additional components such as a heater, a cooler and/or a carbon-dioxide supply. The heater may for example comprise one or more heating elements and may be configured to heat some or all of the dissociation volumes and/or the reservoir 116, e.g. to adjust a temperature during the chemical dissociation/enzymatic digestion of the sample objects. The cooler may for example comprise one or more cooling elements (e.g. a thermoelectric cooling element such as a Peltier element) and may e.g. be configured to cool the reservoir 116, for example for cooling an enzymatic solution contained therein while the device 300 is not in use. The carbon-dioxide supply may be configured to supply carbon-dioxide to the dissociation unit 114, e.g. to some or all of the dissociation volumes and/or to the reservoir 116, e.g. to adjust a carbon dioxide concentration during the chemical dissociation/enzymatic digestion of the sample objects.

In addition to the reservoir 116 for dissolving/enzymatic solutions, the dissociation and filtration unit 108/114 may comprise one or more additional reservoirs, e.g. to supply a buffer or carrier fluid, such as PBS, to supply a cleaning solution for cleaning the device 300 or a part thereof and/or to supply a measurement fluid, such as a viscoelastic fluid (e.g. PBS containing a shear-thinning polymer such as poly(ethylene oxide) (PEO)). The dissociation and filtration unit 108/114 may further comprise a mixing unit, wherein the mixing unit may for example be configured to mix fluids from the reservoir 116 and/or from the additional reservoirs.

The microfluidic device 300 further comprises a control unit 304 arranged between the dissociation and filtration unit 108/114 and the measurement volume 110. The control unit 304 may for example be used to perform control measurements, e.g. optical and/or electrical control measurements, on the sample objects, for example to determine an object count (e.g. the number or density of sample objects and/or cells in a given batch) and/or a cell viability measure.

The microfluidic device 300 further comprises a preparatory unit 306 arranged between the dissociation and filtration unit 108/114 and the measurement volume 110. The preparatory unit 306 may for example comprise one or more filters similar to the sorting unit 108 and/or the filters 204 of the microfluidic devices 200A, 200B, e.g. a first filter 204A such as a cross-flow cell filter for directing larger sample objects to a waste reservoir and/or for exchanging the carrier fluid and/or the dissolving solution (e.g. by a measurement fluid) and a second filter 204B such as a dead-end cell filter downstream of the first filter 204A to prevent clogging of the measurement volume as illustrated in Fig. 3. The preparatory unit 306 may further comprise a measurement fluid inlet for supplying a measurement fluid, e.g. in addition to the carrier fluid and/or the dissolving/enzymatic solution and/or to replace the carrier fluid and/or the dissolving/enzymatic solution prior to performing measurements on the sample objects (for example to adjust a density of sample objects and/or to provide a viscoelastic substance).

The microfluidic device 300 also comprises one or more sheath fluid inlets arranged upstream of and/or in the measurement volume. The sheath fluid inlets may be configured to generate one or more sheath flows (e.g. a pair of sheath flows for confinement along a single direction or two pairs of sheath flows for confinement along two orthogonal directions) for hydrodynamic focusing of the sample objects in the measurement volume.

The microfluidic device 300 further comprises a post-processing unit 308 downstream of the measurement volume 110. The post-processing unit 308 may for example comprise one or more outlets similar to the outlet 104B of the microfluidic device 100 of Fig. 1 and/or one or more reservoirs for collecting sample objects after having performed a measurement thereon in the measurement volume 110, e.g. for further analyses. In some embodiments, the post-processing unit 308 may also comprise a sorter, which may for example be configured to sort the sample objects based on a measurement performed in the measurement volume 110, e.g. for sorting the sample objects by cell type. Additionally or alternatively, the post-processing unit 308 may for example comprise a filter for exchanging the carrier fluid or measurement fluid, e.g. by a cell culture medium.

In some embodiments, some or all of the components of the microfluidic device 300 described above may not be part of the microfluidic device 300, but may instead be integrated into a system for analyzing a sample comprising one or more sample objects containing biological cells according to the invention, e.g. the system 600 of Fig. 6 described below.

Fig. 4 depicts schematic illustrations (not to scale) of sample collection units for a microfluidic device such as the microfluidic devices 100, 200A, 200B and 300 in accordance with exemplary embodiments of the invention. One or more of these sample collections units may be permanently integrated into the microfluidic device. Additionally or alternatively, the one or more of these sample collection units may be configured to be removably connected to the microfluidic device, e.g. as single-use plug-in units.

Panel (a) of Fig. 4 depicts a sample collection unit configured for receiving liquid samples such as a body fluid sample. The sample collection unit comprises a connector that is configured to be coupled to a sample collection device such as a syringe and/or a tube for transferring a sample (e.g. a sample fluid) contained in the sample collection device to the insertion volume of the microfluidic device. The sample collection unit further comprises a flow pump for generating a flow through the insertion volume to transport sample objects from the insertion volume to the sorting unit.

Panel (b) of Fig. 4 depicts a sample collection unit configured for receiving solid samples such as a biopsy sample and/or an aspiration sample. The sample collection unit comprises an opening that is configured to receive a needle such as an aspiration needle (e.g. a fine-needle aspiration (FNA) needle) and/or a biopsy needle (e.g. a fine-needle biopsy (FNB) needle). The opening is in fluid communication with the insertion volume for transferring a sample from the needle to the insertion volume. The sample collection unit further comprises a wiper for wiping off the sample from the needle as illustrated in panel (a) of Fig. 5 (left plot). The wiper may for example be arranged on a side wall of the insertion volume as shown in Fig. 4. The sample collection unit further comprises a flow pump that is configured to generate a flow through the insertion volume to transport sample objects from the insertion volume to the sorting unit as illustrated in panel (a) of Fig. 5 (right plot). The flow pump is further configured to detach a sample from the needle and/or from the wiper by flushing the insertion volume as illustrated in panel (a) of Fig. 5 (center plot).

Panel (c) of Fig. 4 depicts another example of a sample collection unit that is configured for receiving liquid as well as solid samples from a sample collection device such as a syringe, a spatula or a needle. The sample collection unit comprises a collection tube such as a standard centrifuge tube and a transfer pump for transferring a sample from the collection tube to the insertion volume. The transfer pump may for example be mounted on or in a movable head, wherein the moveable head may be configured to insert an inlet of the transfer pump into the collection tube and into the insertion volume or into an inlet in fluid communication with the insertion volume. The transfer pump may be configured to suck the sample from the collection tube and to release the sample into the insertion volume, cf. panel (b) of Fig. 5 (right plot). The transfer pump may also be configured to flush the collection tube, e.g. for detaching a sample from a sample collection device, cf. panel (b) of Fig. 5 (left plot). In some embodiments, the collection tube may comprise a wiper for wiping of a sample from a sample collection device, cf. panel (b) of Fig. 5 (center plot).

In some embodiments, the sample collection unit may comprise two or more of the means for receiving a sample illustrated in panels (a), (b) and (c) of Fig. 4, e.g. the connector of panel (a) as well as the needle opening of panel (b) and/or the collection tube of panel (c).

Fig. 6 shows a schematic illustration (not to scale) of a system 600 for analyzing a sample comprising one or more sample objects containing biological cells according to an exemplary embodiment of the invention. The system 600 is configured for use with a microfluidic device 602 according to any one of the embodiments described herein, for example any one of the microfluidic devices 100, 200A, 200B and 300 described above. In some embodiments, the system may comprise the microfluidic device 600.

The system 600 comprises a mount 604 that is configured to receive the microfluidic device 602 and to hold the microfluidic device 602 in place. The mount 604 may further be configured to provide fluid, electrical and/or optical connections (not shown) to the microfluidic device 602.

The system 600 further comprises a microfluidics unit 606 that is configured to generate fluid flows within the microfluidic device 602, in particular to generate a flow of a carrier fluid through the insertion volume 106 of the microfluidic device 602 to transport sample objects from the insertion volume 106 to the sorting unit (not shown) of the microfluidic device 602 and further on to the dissociation unit 114 and the measurement volume 110 of the microfluidic device 602. To generate and control the fluid flows, the microfluidic device 602 may comprise one or more pumps, one or more valves and/or one or more reservoirs for storing fluids (not shown). Some or all of the pumps, valves and/or reservoirs of the microfluidic devices 100, 200A, 200B and 300 described above may be part of the microfluidics unit 606 instead of being integrated into the respective microfluidic device. The microfluidics unit 606 may for example comprise a reservoir and/or a pump (not shown) for providing a buffer or carrier fluid to the inlet 104A of the microfluidic device 602, e.g. similar to the sample collection unit 302 of the microfluidic device 300. Additionally or alternatively, the microfluidics unit 606 may comprise a reservoir and/or a pump (not shown) for supplying a dissolving solution, for example an enzymatic solution, to the microfluidic device 602 via an inlet 104C, e.g. to the reservoir 116 of the microfluidic devices 100, 200A, 200B and/or directly to a dissociation volume such as the dissociation volume 114A of the microfluidic device 100.

The system 600 comprises a measurement device 608 configured to perform measurements on single cells and/or cell aggregates in the measurement volume 110 of the microfluidic device 602. In the example of Fig. 6, the measurement device 608 is a quantitative phase-contrast microscope, namely a digital holographic microscope (DHM) that is configured to take phase shift images as well as amplitude or intensity images of single cells and/or cell aggregates in the measurement volume 110 of the microfluidic device 602.

The digital holographic microscope 608 comprises an imaging system with an objective 608A, a holographic imaging system 608B and an imaging lens 608C. The imaging system is configured to image a focal plane of the imaging system onto a camera 608D, e.g. through a detection window (not shown) of the microfluidic system 602. The microscope 608 may further comprises an illumination source (not shown) for illuminating the measurement volume 110.

The holographic imaging system 608B is configured to create an interference image on the camera 608D, e.g. by interfering an imaging beam with a reference beam on the camera 608D. The imaging beam may for example be a beam that passes through the measurement volume 110 and propagates from the focal plane of the imaging system to the camera 608D along a first optical path through the holographic imaging system 608B. The reference beam may for example be a beam propagating to the camera 608D along a second optical path through the holographic imaging system 608B. In some examples, the reference beam may be split from the imaging beam, e.g. using a beam splitter or a diffraction grating, i.e. the reference beam may also have passed through the measurement volume 110 and may propagate to the camera 608D from the focal plane of the imaging system along the second optical path. In other examples, the reference beam may not have passed through the measurement volume 110 and may e.g. be split from the imaging beam in front of the measurement volume 110.

The digital holographic microscope 608 may be an on-axis digital holographic microscope, in which the imaging beam and the reference beam propagate along the same axis when interfering, i.e. interfere at an angle of 0°. The digital holographic microscope 608 may for example be configured to extract or reconstruct a phase shift image as well as an intensity image of the sample from a plurality of interference images, e.g. by varying a phase offset between the reference and imaging beams using the holographic imaging system 608B. Preferably, the microscope 608 is an off-axis digital holographic microscope, in which the imaging beam and the reference beam interfere under an angle. In this case, the digital holographic microscope 608 may be configured to extract or reconstruct the phase shift image as well as the intensity image of the sample from a single interference image.

In other embodiments, the measurement device 608 may comprise a different type of microscope, e.g. a bright-field microscope and/or a fluorescence microscope, instead of or in addition to the digital holographic microscope. Additionally or alternatively, the measurement device 608 may also be configured to perform other types of optical measurements, e.g. spectroscopic measurements, and/or electrical measurements such as impedance measurements.

The system 600 further comprises a controller 610 that is configured to control the microfluidics unit 606, the measurement device 608, the sorting unit of the microfluidic device 602 and/or the dissociation unit 114 of the microfluidic device 602, e.g. by generating corresponding analog and/or digital control signals. The controller 610 may be implemented in hardware, software or a combination thereof. The controller 610 may for example comprise a processing device and a memory or storage medium storing instructions for execution by the processing device to provide the functionality described herein. The controller 306 may for example comprise a central processing unit (CPU), a graphics processing unit (GPU), a field programmable gate array (FPGA), an application-specific integrated circuit (ASIC), and/or a microcontroller.

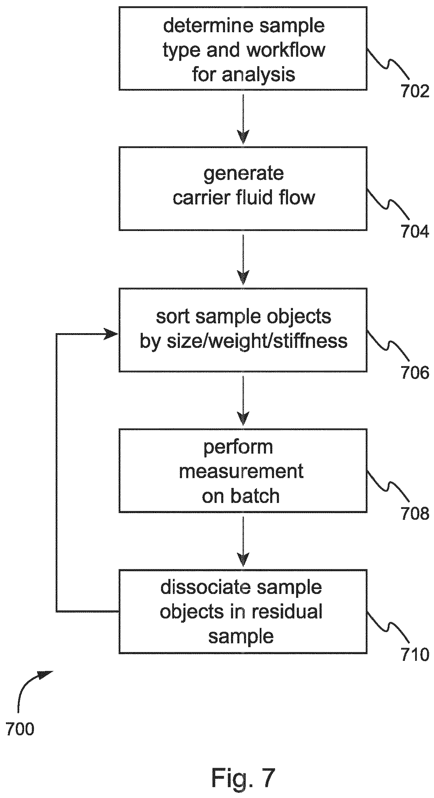

The controller 610 is configured to execute a method of analyzing a sample comprising one or more sample objects containing biological cells according to any one of the embodiments described herein by controlling the microfluidics unit 606, the measurement device 608, the sorting unit of the microfluidic device 602 and/or the dissociation unit 114 of the microfluidic device 602 accordingly. The controller 610 may in particular be configured to execute some or all of the steps of the method 700 of Fig. 7 described below.

In some embodiments, the system 600 may comprise one or more means for dissociating sample objects, wherein the controller 610 may be configured to control said means. The system 600 may for example comprise one or more reservoirs for supplying a dissolving solution, for example an enzymatic solution, to the dissociation unit of the microfluidic device 602, e.g. as described above. The system 600 may also comprise an acoustic source, in this example an ultrasound source 612, that is configured to apply acoustic waves/ultrasound to sample objects in the dissociation unit of the microfluidic device 602. The ultrasound source 612 may for example comprise a sonotrode that is configured to apply ultrasound to a passive ultrasound emitter of the microfluidic device 602 such as the ultrasound emitter 116D of the microfluidic device 200B of Fig. 2b. The system 600 may also comprise a magnetic stirrer that is configured to move a magnetic stir bar in the microfluidic device, e.g. similar to the magnetic stirrer 116C of the microfluidic device 200B of Fig. 2b. In some embodiments, the ultrasound source or a part thereof and/or the magnetic stirrer or a part thereof may be integrated into the mount 602.

Fig. 7 depicts a flow chart of a method 700 of analyzing a sample comprising one or more sample objects containing biological cells according to an exemplary embodiment of the invention. The method 700 is executed using a microfluidic device according to any one of the embodiments described herein. The method 700 may for example be executed using a microfluidic device similar to the microfluidic devices 200A, 200B of Figs. 2a, 2b that contains the four dissociation volumes 114A-D and the means 116, 116A-D for dissociating sample objects and the system 600 of Fig. 6, which are used as examples for illustration purposes in the following. This is, however, not intended to be limiting in any way and the method 700 may also be implemented using a microfluidic device such as the microfluidic device 100, 300 or 602 and/or may be implemented without the system 600 or with a different system for analyzing a sample comprising one or more sample objects containing biological cell according to any one of the embodiments described herein. Furthermore, the method 700 is not limited to the order of execution shown in the flowchart of Fig. 7. As far as technically feasible, the method 700 may be executed in an arbitrary order and parts thereof may be executed simultaneously at least in part, e.g. steps 708 and 710.

The method 700 may be used for analyzing a wide range of samples comprising sample objects such as single cells 102A, cell aggregates 102B and/or one or more pieces of continuous tissue material 102C, for example biopsy samples, aspiration samples, body fluid samples and/or smear samples.

The method 700 comprises, in step 702, determining a sample type of the sample 102, wherein the sample type characterizes one or more of a procedure used for obtaining the sample 102 from a patient (e.g. biopsy, aspiration, puncture, lavage or smear), a body part that the sample 102 was obtained from (e.g. an organ, a type of tissue or a type of body fluid), a size of the sample objects 102A-C in the sample 102 and a weight of the sample objects 102A-C in the sample 102. The sample type may for example be entered manually by a user and/or may be determined by scanning a label such as a barcode associated with the sample 102 and/or with a sample collection device.

Based on the sample type, a workflow for analyzing the sample 102 is determined. This may in particular comprise choosing a number of dissociation steps that are to be performed, means for dissociating sample objects that are to be used (e.g. selecting a dissolving solution that is to be used) and/or parameters for the dissociation of the sample objects such as a duration or a concentration of enzymes.

The sample 102 may be provided in the insertion volume 106 of the microfluidic device 200A/200B prior to execution of the method 700 or as part of the method 700, e.g. as illustrated in Figs. 4 and 5. This may also comprise providing a buffer or carrier fluid such as PBS in the insertion volume 106 for suspending the sample objects 102A-C therein. Providing the sample 102 may comprise flushing the insertion volume 106 and/or a collection tube for detaching the sample 102 from a sample collection device, such as a biopsy needle, and/or may comprise wiping off the sample 102 from the sample collection device at a wiper.

In step 704, a flow of the carrier fluid is generated through the insertion volume 106 to transport sample objects 102A-C from the insertion volume 106 to the sorting unit 108. In step 706, the sample objects 102A-C pass through the sorting unit 108. The sorting unit 108 sorts the sample objects 102A-C by size, weight and/or stiffness into a first batch of sample objects, which may e.g. comprise some or all of the sample objects 102A-C having a size smaller than a sorting threshold (e.g. smaller than 40 µm), and a first residual sample, which may contain the remaining sample objects 102A-C that are not contained in the first batch. The first residual sample may in particular contain the sample objects 102A-C having a size larger than the sorting threshold.

The first batch of sample objects is then transferred to the measurement volume 110 via the first outlet 108A of the sorting unit 108 and a first measurement is performed on the sample objects of the first batch in step 708. The first measurement may for example comprise taking one or more phase shift images of the sample objects in the first batch using the digital holographic microscope 608 of the system 600.

Prior to performing the first measurement, a density of the sample objects 102A-C in the first batch may be determined (e.g. number of sample objects per volume or a mean inter-object distance) and may optionally be adjusted, e.g. by adding more fluid, for example to ensure that the density is sufficiently low such that separate sample objects 102A-C can easily be discerned in the phase shift images. Before entering the measurement volume 110, the first batch of sample objects 102A-C may also be filtered using the filters 204, e.g.to prevent larger sample objects that might clog the measurement volume 110 from entering the measurement volume 110.

For taking the phase shift images, the sample objects 102A-C in the first batch may be focused hydrodynamically and/or viscoelastically in a focal plane of the microscope 608, for example by generating one or more sheath flows as described above. Preferably, the sample objects 102A-C are focused viscoelastically in the measurement volume 110 by performing the measurement in a viscoelastic fluid, e.g. by using a viscoelastic carrier fluid, by adding a viscoelastic measurement fluid to the carrier fluid prior to performing the measurement and/or by replacing the carrier fluid with a viscoelastic measurement fluid prior to performing the measurement. The viscoelastic fluid may comprise one or more shear-thinning polymers, which may for example be selected from the group consisting of poly(ethylene oxide) (PEO), poly(vinyl pyrrolidone) (PVP), hyaluronic acid (HA) and polyacrylamide (PAA). Preferably, the shear-thinning polymer is poly(ethylene oxide) (PEO), which may e.g. be dissolved in a buffer such as PBS. The shear-thinning polymer may have a molecular weight between 2 MDa and 10 MDa, preferably between 3 MDa and 6 MDa, most preferably between 3.5 MDa and 4.5 MDa, in one example of 4.0 MDa. A mass fraction of the shear-thinning polymer in the viscoelastic fluid may be less than 0.2%, preferably between 0.03% and 0.12%, most preferably between 0.04% and 0.06%, in one example 0.05%. Such a viscoelastic fluid may allow for reliable and accurate viscoelastic focusing of sample objects of different sizes such as single cells and cell aggregates at low flow velocities, thus reducing mechanical stress on the sample objects, which may damage the sample objects and/or influence the measurement. Furthermore, the inventors have found that such a viscoelastic fluid does not induce a formation of artificial cell aggregates even in blood samples, which may for example be prone to polymer-induced platelet aggregation.

The first residual sample is transferred to the dissociation unit 114 via the second outlet 108B of the sorting unit 108 for dissociating the sample objects 102A-C contained therein into single cells and/or cell aggregates at least in part in step 710. The sample objects 102A-C may be dissociated using any combination of means for dissociating sample objects described above, for example by chemical dissociation (e.g. enzymatic digestion) and/or mechanical dissociation.

In some embodiments, the means for dissociating the sample objects 102A-C in the first residual sample may be selected based on the sample type determined in step 702. Using the means 118A-D for controlling fluid flows through the dissociation volumes 114A-D, the sample objects 102A-C may be selectively directed to the respective dissociation volume. For example, if the sample is expected to contain larger pieces of tissue 102C (e.g. if the sample is a biopsy sample), the magnetic stirrer 116C and/or the blades 116A may be used for cutting the pieces of tissue 102C into smaller pieces. On the other hand if the sample is not expected to contain any larger pieces of tissue 102C (e.g. if the sample is a body fluid sample), a dissolving solution and/or the constricted portions 116B may be used to dissociate larger cell aggregates 102B into single cells and/or smaller cell aggregates. Furthermore, parameters such as an enzyme concentration and/or a duration of the dissociation step (e.g. a duration of exposure to the enzyme solution and/or a duration for which the magnetic stirrer 116C is activated) may be chosen based on the sample type.

The method 700 may then return to step 706 for sorting the dissociated sample objects 102A-C from the first residual sample into a second batch of sample objects and a second residual sample, e.g. using the sorter 202 or the sorting unit 108, as described above for the first batch of sample objects and the first residual sample. The method 700 may then proceed to step 708 to transfer the second batch of sample objects to measurement volume 110 for performing a second measurement on the dissociated sample objects in the second batch, e.g. as described above for the first batch of sample objects.

The second residual sample may either be discarded or may be used to obtain one or more additional batches of sample objects by repeatedly performing steps 706 to 710, i.e. by dissociating the sample objects in a residual sample separated from a previous batch using the dissociation unit 114 and subsequently sorting the dissociated sample objects into the respective additional batch of sample objects and a new residual sample using the sorter 202. In some embodiments, different means for dissociating sample objects may be used for different dissociation steps. For example, the magnetic stirrer 116C may be used during the first dissociation step (i.e. for dissociating sample objects 102A-C in the first residual sample), the blades 116A may be used during the second dissociation step (i.e. for dissociating sample objects 102A-C in the second residual sample), the ultrasound emitter 116D may be used during the third dissociation step (i.e. for dissociating sample objects 102A-C in the third residual sample) and the constricted portions 116B may be used during the fourth dissociation step (i.e. for dissociating sample objects 102A-C in the fourth residual sample). Additionally or alternatively, the sample objects 102A-C may be exposed to a dissolving solution during some or all of the dissociation steps. In some examples, different dissolving/enzymatic solutions may be used in different dissociations steps, e.g. a first solution in the first dissociation step and a second solution in the second dissociation step.

The number of dissociations steps that are performed may be determined or chosen based on the sample type determined in step 702. For example, a single dissociation step may be performed if the sample type indicates that the sample is a body fluid sample and/or if the sample type indicates that the sample is a smear sample, whereas multiple dissociation steps may be performed if the sample type indicates that the sample is a biopsy sample and/or if the sample type indicates that the sample is an aspiration sample.

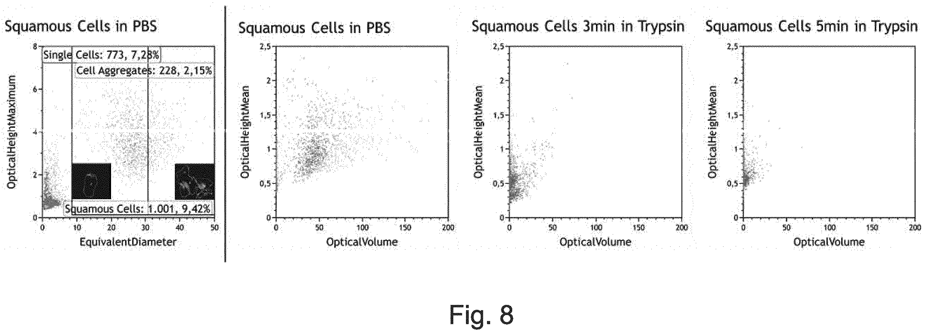

Fig. 8 shows experimental data demonstrating the sorting and dissociation of sample objects containing biological cells using a microfluidic device according to the invention. For this, samples comprising squamous cells from an oral smear of a patient were analyzed after sorting and dissociating sample objects using different dissociation protocols (center-right and right plots) and compared to a reference sample for which no sorting or dissociation was performed (left and center-left plots). Phase shift images of the sample objects were obtained with a digital holographic microscope. The equivalent diameter, the maximum optical height, the mean optical height and the optical volume of the sample objects were extracted from the phase shift images and are shown in Fig. 8. Dissolving the sample objects using an enzymatic solution containing trypsin causes cell death and destruction of squamous cells, leading to a substantial decrease in the mean optical height and optical volume of the sample objects and thereby the conclusion of trypsin as unsuitable dissociation mean for squamous cells (center-left plot: no dissociation, center-right plot: treatment with trypsin for 3 min, right plot: treatment with trypsin for 5 min).

The embodiments of the present invention disclosed herein only constitute specific examples for illustration purposes. The present invention can be implemented in various ways and with many modifications without altering the underlying basic properties. Therefore, the present invention is only defined by the claims as stated below.

### LIST OF REFERENCE SIGNS

100 - microfluidic device
102 - sample
102A - single cell
102B - cell aggregate
102C - piece of continuous tissue material
104 - substrate
104A - inlet
104B - outlet
104C - inlet for dissolving/enzymatic solution
106 - insertion volume
108 - sorting unit
108A - first outlet of sorting unit 108
108B - second outlet of sorting unit 108
108C - filter
110 - measurement volume
112 - optical detection window
114 - dissociation unit
114A-D - dissociation volumes
116 - reservoir for dissolving/enzymatic solution
116A - static obstacles (blades)
116B - constricted portions
116C - magnetic stirrer
116D - acoustic/ultrasound emitter
118A-D - pumps/valves
200A, 200B - microfluidic device
202 - sorter
202A - first outlet of sorter 202
202B - second outlet of sorter 202
204, 204A, 204B - filter
300 - microfluidic device
302 - sample collection unit
304 - control unit
306 - preparatory unit
308 - post-processing unit
600 - system for analyzing a sample comprising sample objects containing biological cells
602 - microfluidic device
604 - mount
606 - microfluidics unit
608 - measurement device (digital holographic microscope)
608A - objective
608B - holographic imaging system
608C - imaging lens
608D - camera
610 - controller
612 - acoustic/ultrasound source
700 - method for analyzing a sample comprising sample objects containing biological cells
702 - step of determining sample type
704 - step of generating carrier fluid flow
706 - step of sorting sample objects by size, weight and/or stiffness
708 - step of performing measurement on a batch of sorted sample objects
710 - step of dissociating sample objects in a residual sample of sorted sample objects

## Claims

1. A microfluidic device (100, 200A, 200B, 300, 602) for analyzing a sample (102) comprising one or more sample objects (102A-C) containing biological cells, wherein the one or more sample objects (102A-C) comprise one or more single cells (102A), one or more cell aggregates (102B) and/or one or more pieces of continuous tissue material (102C), the microfluidic device (100, 200A, 200B, 300, 602) comprising:
an insertion volume (106) configured to receive the sample (102);
a sorting unit (108) having an inlet that is in fluid communication with the insertion volume (106), a first outlet (108A) and a second outlet (108B), wherein the sorting unit (108) is configured to sort the sample objects (102A-C) by size, weight and/or stiffness by directing sample objects (102A-C) having a smaller size, a smaller weight and a lower stiffness, respectively, from the inlet towards the first outlet (108A) and sample objects (102A-C) having a larger size, a larger weight and a higher stiffness, respectively, from the inlet towards the second outlet (108B);
a measurement volume (110) that is in fluid communication with the first outlet (108A) of the sorting unit (108); and
a dissociation unit (114) comprising means (116, 116A-D) for dissociating sample objects (102A-C) into single cells and/or cell aggregates at least in part, wherein an inlet of the dissociation unit (114) is in fluid communication with the second outlet (108B) of the sorting unit (108) and an outlet of the dissociation unit (114) is in fluid communication with an inlet of the measurement volume (110).

2. The microfluidic device (100, 200A, 200B, 300, 602) of claim 1, wherein the dissociation unit (114) comprises a dissociation volume (114A-D) and the means (116, 116A-D) for dissociating sample objects (102A-C) comprise one or more of:
a reservoir (116) configured to supply a dissolving solution to the dissociation volume (114A-D), the dissolving solution comprising one or more chemical and/or biochemical substances for chemically dissolving sample objects (102A-C), in particular one or more enzymes for dissolving sample objects (102A-C) by enzymatic digestion;
an acoustic emitter, in particular an ultrasound emitter (116D), configured to apply acoustic waves to sample objects (102A-C) in the dissociation volume (114D);
one or more static obstacles (116A) arranged in the dissociation volume (114A) configured to mechanically dissociate sample objects (102A-C) colliding with the one or more obstacles (116A);
one or more curved and/or constricted portions (116B) of the dissociation volume (114B) configured to dissociate sample objects (102A-C) by generating a shear force on sample objects (102A-C) flowing through the dissociation volume (114B);
a stirrer (116C) configured to move a stir bar in the dissociation volume (114C) for dissociating sample objects (102A-C); and
one or more moveable elements configured to cut and/or grind sample objects (102A-C) in the dissociation volume (114A-D),
wherein preferably the dissociation unit (114) comprises a plurality of dissociation volumes (114A-D), each of which is associated with a different means for dissociating sample objects, in particular wherein the dissociation unit (114) further comprises a plurality of pumps (118A-D) and/or valves (118A-D) for selectively directing sample objects (102A-C) into one of the dissociation volumes (114A-D) and/or wherein the sorting unit (108) is configured to selectively direct sample objects (102A-C) into one of the dissociation volumes (114A-D) based on a size, a weight and/or a stiffness of the respective sample object (102A-C).

3. The microfluidic device (100, 200A, 200B, 300, 602) of claim 1 or 2, wherein the sorting unit (108) comprises one or more of an inertia-based cell filter, an acoustic cell filter, a hydrodynamic cell filter, a viscoelastic filter, a density sorter, a field-flow fractionation filter, a dielectrophoretic filter, a deterministic lateral displacement filter, a weir filter, a dead-end cell filter, a cell-stiffness sorter and a cross-flow cell filter.

4. The microfluidic device (100, 200A, 200B, 300, 602) of any one of the preceding claims, wherein:
the outlet of the dissociation unit (114) is in fluid communication with the measurement volume (110) via the sorting unit (108); and/or
the outlet of the dissociation unit (114) further comprises a sorter (202) having a first outlet (202A) that is in fluid communication with the measurement volume (110) and a second outlet (202B) that is in fluid communication with the inlet of the dissociation unit (114), wherein the sorter (202) is configured to sort the sample objects (102A-C) by size, weight and/or stiffness by directing sample objects (102A-C) having a smaller size, a smaller weight and a lower stiffness, respectively, towards the first outlet (202A) and sample objects (102A-C) having a larger size, a larger weight and a higher stiffness, respectively, towards the second outlet (202B); and/or
the microfluidic device (100, 200A, 200B, 300, 602) further comprises a filter (204) arranged between the outlet of the dissociation unit (114) and the inlet of the measurement volume (110), wherein the filter (204) is configured to prevent sample objects (102A-C) having a size larger than a filtering threshold from entering the measurement volume (110).

5. The microfluidic device (100, 200A, 200B, 300, 602) of any one of the preceding claims, wherein:
the microfluidic device (100, 200A, 200B, 300, 602) comprises a sample collection unit (302) comprising one or more of a connector configured to couple a syringe and/or a tube containing a sample comprising the one or more sample objects (102A-C) to the insertion volume (106); an opening configured to receive an aspiration needle and/or a biopsy needle, wherein the opening is in fluid communication with the insertion volume (106); and a transfer pump configured to transfer a sample from a collection tube to the insertion volume (106); and/or
the microfluidic device (100, 200A, 200B, 300, 602) further comprises a wiper for wiping off a sample (102) from a sample collection device, in particular for wiping off a biopsy sample from a biopsy needle; and/or themicrofluidic device (100, 200A, 200B, 300, 602) further comprises a flow pump configured to generate a flow through the insertion volume (106) to transport the sample objects (102A-C) from the insertion volume (106) to the sorting unit (108), in particular wherein the flow pump is configured to detach a sample (102) from a sample collection device and/or from the wiper by flushing and/or sucking out the insertion volume (106).

6. The microfluidic device (100, 200A, 200B, 300, 602) of any one of the preceding claims, wherein the measurement volume (110) comprises a first measurement channel and a second measurement channel connected in parallel to the first measurement channel, the second measurement channel having a larger cross-sectional area than the first measurement channel, in particular wherein the microfluidic device (100, 200A, 200B, 300, 602) comprises a pump and/or a valve for selectively directing sample objects (102A-C) into either the first measurement channel or the second measurement channel and/or wherein the sorting unit (108) is configured to selectively direct sample objects (102A-C) into either the first measurement channel or the second measurement channel based on a size, a weight and/or a stiffness of the respective sample object (102A-C).

7. The microfluidic device (100, 200A, 200B, 300, 602) of any one of the preceding claims, wherein:
the microfluidic device (100, 200A, 200B, 300, 602) further comprises a measurement fluid inlet arranged between the outlet of the dissociation unit (114) and the inlet of the measurement volume (110) for supplying a measurement fluid; and/or
the measurement volume (110) comprises an optical detection window (112) for taking microscopic images, in particular phase shift images of sample objects (102A-C) in the measurement volume (110).

8. A method (700) of analyzing a sample (102) comprising one or more sample objects (102A-C) containing biological cells using a microfluidic device (100, 200A, 200B, 300, 602) according to any one of the preceding claims, wherein the one or more sample objects (102A-C) comprise one or more single cells (102A), one or more cell aggregates (102B) and/or one or more pieces of continuous tissue material (102C), the method (700) comprising:
generating a flow of a carrier fluid through the insertion volume (106) to transport sample objects (102A-C) from the insertion volume (106) to the sorting unit (108);
sorting the sample objects (102A-C) with the sorting unit (108) into a first batch of sample objects and a first residual sample, wherein the first residual sample comprises sample objects (102A-C) having a larger size, a larger weight and/or a higher stiffness than the sample objects (102A-C) in the first batch;
performing a first measurement on the sample objects (102A-C) of the first batch in the measurement volume (110);
dissociating sample objects (102A-C) in the first residual sample with the dissociation unit (114) into single cells and/or cell aggregates at least in part to obtain a second batch of sample objects; and
performing a second measurement on the dissociated sample objects (102A-C) in the second batch in the measurement volume (110).

9. The method (700) of claim 8, wherein the second batch of sample objects is obtained by sorting the dissociated sample objects (102A-C) into the second batch of sample objects and a second residual sample, wherein the second residual sample comprises sample objects (102A-C) having a larger size, a larger weight and/or a higher stiffness than the sample objects (102A-C) in the second batch, wherein the method (700) preferably further comprises obtaining one or more additional batches of sample objects by:
dissociating sample objects (102A-C) in a residual sample separated from a previous batch into single cells and/or cell aggregates at least in part using the dissociation unit (114); and
sorting the dissociated sample objects (102A-C) into the respective additional batch of sample objects and a new residual sample, wherein the new residual sample comprises sample objects (102A-C) having a larger size, a larger weight and/or a higher stiffness than the sample objects (102A-C) in the respective additional batch.

10. The method (700) of claim 8 or 9, wherein:
the method further comprises determining a sample type of the sample (102), wherein the sample type characterizes one or more of a procedure used for obtaining the sample (102) from a patient, a body part that the sample (102) was obtained from, a size of the sample objects (102A-C) in the sample (102) and a weight of the sample objects (102A-C) in the sample (102); determining a number of dissociation steps that are to be performed based on the sample type; and performing the dissociation steps and sorting the dissociated sample objects (102A-C) by size, weight and/or stiffness to obtain a plurality of batches of sample objects; and/or
the sample (102) comprises a biopsy sample, a resection sample, an aspiration sample, a body fluid sample and/or a smear sample,
in particular wherein no dissociation step or a single dissociation step is performed if the sample type indicates that the sample (102) is a body fluid sample and/or if the sample type indicates that the sample (102) is a smear sample and multiple dissociation steps are performed if the sample type indicates that the sample (102) is a biopsy sample, if the sample type indicates that the sample is a resection sample and/or if the sample type indicates that the sample (102) is an aspiration sample.

11. The method (700) of any one of claims 8 to 10, wherein:
the dissociation unit (114) of the microfluidic device (100, 200A, 200B, 300, 602) comprises a plurality of means (116, 116A-D) for dissociating the sample objects (102A-C) and the method (700) further comprises selecting a means for dissociating the sample objects from the plurality of means (116, 116A-D) for dissociating the sample objects (102A-C) based on the sample type; and/or
the method (700) further comprises determining an object count and/or a cell viability measure for one or more of the batches of sample objects by optical, electrical and/or mechanical means, in particular wherein the object count is determined for one or more of the batches and the method (700) further comprises adjusting a density of sample objects (102A-C) in the respective batch based on the determined object count by supplying a measurement fluid.

12. The method (700) of any one of claims 8 to 11, wherein:
performing the first measurement and/or performing the second measurement comprises or corresponds to taking an image of the sample objects (102A-C) in the measurement volume (110) using a microscope, in particular wherein performing the first measurement and/or performing the second measurement comprises or corresponds to taking a phase shift image of the sample objects (102A-C) in the measurement volume (110) using a phase-contrast microscope (608); and/or
performing the first measurement and/or performing the second measurement comprises hydrodynamically and/or viscoelastically focusing the respective sample objects (102A-C) in the measurement volume (110).

13. A system (600) for analyzing a sample (102) comprising one or more sample objects (102A-C) containing biological cells, the one or more sample objects (102A-C) comprising one or more single cells (102A), one or more cell aggregates (102B) and/or one or more pieces of continuous tissue material (102C), wherein the system (600) is configured for use with a microfluidic device (100, 200A, 200B, 300, 602) according to any one of claims 1 to 7 and comprises:
a microfluidics unit (606) configured to generate a flow of a carrier fluid through the insertion volume (106) of the microfluidic device (100, 200A, 200B, 300, 602) to transport sample objects (102A-C) from the insertion volume (106) to the sorting unit (108) of the microfluidic device (100, 200A, 200B, 300, 602);
a measurement device (608) configured to perform measurements on single cells and/or cell aggregates in the measurement volume (110) of the microfluidic device (100, 200A, 200B, 300, 602); and
a controller (610) configured to control the microfluidics unit (606), the measurement device (608), the sorting unit (108) of the microfluidic device (100, 200A, 200B, 300, 602) and/or the dissociation unit (114) of the microfluidic device (100, 200A, 200B, 300, 602) to execute a method (700) according to any one of claims 8 to 12.

14. The system (600) of claim 13, wherein the measurement device (608) is or comprises a phase-contrast microscope, in particular a digital holographic microscope (608A-D), configured to take phase shift images of single cells and/or cell aggregates in the measurement volume (110) of the microfluidic device (100, 200A, 200B, 300, 602).

15. The system (600) of claim 13 or 14, wherein:
the system (600) further comprises an acoustic source (612), in particular an ultrasound source, that is configured to apply acoustic waves to sample objects (608A-D) in the dissociation unit (114) of the microfluidic device (100, 200A, 200B, 300, 602), wherein the controller (610) is configured to control the acoustic source (612) to dissociate sample objects (102A-C) in the dissociation unit (114) by applying acoustic waves to the sample objects (102A-C); and/or
the microfluidics unit (606) is configured to provide a dissolving solution to the dissociation unit (114) of the microfluidic device (100, 200A, 200B, 300, 602), the dissolving solution comprising one or more chemical and/or biochemical substances for chemically dissolving sample objects (102A-C), in particular one or more enzymes for dissolving sample objects (102A-C) by enzymatic digestion, wherein the controller (610) is configured to control the microfluidics unit (606) to dissociate sample objects (102A-C) in the dissociation unit (114) by supplying the dissolving solution to the dissociation unit (114); and/or
the system (600) further comprises a microfluidic device (100, 200A, 200B, 300, 602) according to any one of claims 1 to 7.

## Patentansprüche

1. Mikrofluidikvorrichtung (100, 200A, 200B, 300, 602) zum Analysieren einer Probe (102), die ein oder mehrere Probenobjekte (102A-C) umfasst, die biologische Zellen enthalten, wobei das eine oder die mehreren Probenobjekte (102A-C) eine oder mehrere einzelne Zellen (102A), ein oder mehrere Zellaggregate (102B) und/oder ein oder mehrere Stücke von kontinuierlichem Gewebematerial (102C) umfassen, wobei die Mikrofluidikvorrichtung (100, 200A, 200B, 300, 602) Folgendes umfasst:
ein Einführvolumen (106), das dazu eingerichtet ist, die Probe (102) aufzunehmen;
eine Sortiereinheit (108) mit einem Einlass, der in Fluidverbindung mit dem Einführvolumen (106) steht, einem ersten Auslass (108A) und einem zweiten Auslass (108B), wobei die Sortiereinheit (108) dazu eingerichtet ist, die Probenobjekte (102A-C) nach Größe, Gewicht und/oder Steifigkeit zu sortieren, indem Probenobjekte (102A-C) mit einer kleineren Größe, einem kleineren Gewicht bzw. einer niedrigeren Steifigkeit von dem Einlass in Richtung des ersten Auslasses (108A) geleitet werden und Probenobjekte (102A-C) mit einer größeren Größe, einem größeren Gewicht bzw. einer höheren Steifigkeit von dem Einlass in Richtung des zweiten Auslasses (108B) geleitet werden;
ein Messvolumen (110), das in Fluidverbindung mit dem ersten Auslass (108A) der Sortiereinheit (108) steht; und
eine Dissoziationseinheit (114), die Mittel (116, 116A-D) umfasst, um Probenobjekte (102A-C) zumindest teilweise in einzelne Zellen und/oder Zellaggregate zu dissoziieren, wobei ein Einlass der Dissoziationseinheit (114) in Fluidverbindung mit dem zweiten Auslass (108B) der Sortiereinheit (108) steht und ein Auslass der Dissoziationseinheit (114) in Fluidverbindung mit einem Einlass des Messvolumens (110) steht.

2. Mikrofluidikvorrichtung (100, 200A, 200B, 300, 602) nach Anspruch 1, wobei die Dissoziationseinheit (114) ein Dissoziationsvolumen (114A-D) umfasst und die Mittel (116, 116A-D) zum Dissoziieren von Probenobjekten (102A-C) eines oder mehrere der Folgenden umfassen:
ein Reservoir (116), das dazu eingerichtet ist, dem Dissoziationsvolumen (114A-D) eine Auflösungslösung zuzuführen, wobei die Auflösungslösung eine oder mehrere chemische und/oder biochemische Substanzen zum chemischen Auflösen von Probenobjekten (102A-C) umfasst, insbesondere ein oder mehrere Enzyme zum Auflösen von Probenobjekten (102A-C) durch enzymatische Verdauung;
einen akustischen Emitter, insbesondere einen Ultraschallemitter (116D), der dazu eingerichtet ist, akustische Wellen auf Probenobjekte (102A-C) in dem Dissoziationsvolumen (114D) anzuwenden;
ein oder mehrere statische Hindernisse (116A), die in dem Dissoziationsvolumen (114A) angeordnet sind und dazu eingerichtet sind, Probenobjekte (102A-C), die mit dem einen oder den mehreren Hindernissen (116A) kollidieren, mechanisch zu dissoziieren;
einen oder mehrere gekrümmte und/oder verengte Abschnitte (116B) des Dissoziationsvolumens (114B), die dazu eingerichtet sind, Probenobjekte (102A-C) durch Erzeugen einer Scherkraft auf Probenobjekte (102A-C), die durch das Dissoziationsvolumen (114B) fließen, zu dissoziieren;
einen Rührer (116C), der dazu eingerichtet ist, einen Rührstab in dem Dissoziationsvolumen (114C) zum Dissoziieren von Probenobjekten (102A-C) zu bewegen; und
ein oder mehrere bewegliche Elemente, die dazu eingerichtet sind, Probenobjekte (102A-C) in dem Dissoziationsvolumen (114A-D) zu schneiden und/oder zu mahlen,
wobei vorzugsweise die Dissoziationseinheit (114) eine Vielzahl von Dissoziationsvolumina (114A-D) umfasst, von denen jedes mit einem anderen Mittel zum Dissoziieren von Probenobjekten assoziiert ist, insbesondere wobei die Dissoziationseinheit (114) ferner eine Vielzahl von Pumpen (118A-D) und/oder Ventilen (118A-D) zum selektiven Leiten von Probenobjekten (102A-C) in eines der Dissoziationsvolumina (114A-D) umfasst und/oder wobei die Sortiereinheit (108) dazu eingerichtet ist, Probenobjekte (102A-C) basierend auf einer Größe, einem Gewicht und/oder einer Steifigkeit des jeweiligen Probenobjekts (102A-C) selektiv in eines der Dissoziationsvolumina (114A-D) zu leiten.

3. Mikrofluidikvorrichtung (100, 200A, 200B, 300, 602) nach Anspruch 1 oder 2, wobei die Sortiereinheit (108) eines oder mehrere von einem trägheitsbasierten Zellfilter, einem akustischen Zellfilter, einem hydrodynamischen Zellfilter, einem viskoelastischen Filter, einem Dichtesortierer, einem Feldflussfraktionierungsfilter, einem dielektrophoretischen Filter, einem deterministischen Lateralverschiebungsfilter, einem Wehrfilter, einem Dead-End-Zellfilter, einem Zellsteifigkeitssortierer und einem Querflusszellfilter umfasst.

4. Mikrofluidikvorrichtung (100, 200A, 200B, 300, 602) nach einem der vorhergehenden Ansprüche, wobei:
der Auslass der Dissoziationseinheit (114) über die Sortiereinheit (108) in Fluidverbindung mit dem Messvolumen (110) steht; und/oder
der Auslass der Dissoziationseinheit (114) ferner einen Sortierer (202) mit einem ersten Auslass (202A), der in Fluidverbindung mit dem Messvolumen (110) steht, und einem zweiten Auslass (202B), der in Fluidverbindung mit dem Einlass der Dissoziationseinheit (114) steht, umfasst, wobei der Sortierer (202) dazu eingerichtet ist, die Probenobjekte (102A-C) nach Größe, Gewicht und/oder Steifigkeit zu sortieren, indem Probenobjekte (102A-C) mit einer kleineren Größe, einem kleineren Gewicht bzw. einer niedrigeren Steifigkeit in Richtung des ersten Auslasses (202A) geleitet werden und Probenobjekte (102A-C) mit einer größeren Größe, einem größeren Gewicht bzw. einer höheren Steifigkeit in Richtung des zweiten Auslasses (202B) geleitet werden; und/oder
die Mikrofluidikvorrichtung (100, 200A, 200B, 300, 602) ferner einen Filter (204) umfasst, der zwischen dem Auslass der Dissoziationseinheit (114) und dem Einlass des Messvolumens (110) angeordnet ist, wobei der Filter (204) dazu eingerichtet ist, zu verhindern, dass Probenobjekte (102A-C) mit einer Größe, die größer als eine Filterschwelle ist, in das Messvolumen (110) eintreten.

5. Mikrofluidikvorrichtung (100, 200A, 200B, 300, 602) nach einem der vorhergehenden Ansprüche, wobei:
die Mikrofluidikvorrichtung (100, 200A, 200B, 300, 602) eine Probensammeleinheit (302) umfasst, die eines oder mehrere der Folgenden umfasst: einen Verbinder, der dazu eingerichtet ist, eine Spritze und/oder ein Röhrchen, das eine Probe enthält, die das eine oder die mehreren Probenobjekte (102A-C) umfasst, mit dem Einführvolumen (106) zu koppeln; eine Öffnung, die dazu eingerichtet ist, eine Aspirationsnadel und/oder eine Biopsienadel aufzunehmen, wobei die Öffnung in Fluidverbindung mit dem Einführvolumen (106) steht; und eine Transferpumpe, die dazu eingerichtet ist, eine Probe von einem Sammelröhrchen zu dem Einführvolumen (106) zu transferieren; und/oder
die Mikrofluidikvorrichtung (100, 200A, 200B, 300, 602) ferner einen Wischer zum Abwischen einer Probe (102) von einer Probensammelvorrichtung, insbesondere zum Abwischen einer Biopsieprobe von einer Biopsienadel, umfasst; und/oder die Mikrofluidikvorrichtung (100, 200A, 200B, 300, 602) ferner eine Flusspumpe umfasst, die dazu eingerichtet ist, einen Fluss durch das Einführvolumen (106) zu erzeugen, um die Probenobjekte (102A-C) von dem Einführvolumen (106) zu der Sortiereinheit (108) zu transportieren, insbesondere wobei die Flusspumpe dazu eingerichtet ist, eine Probe (102) von einer Probensammelvorrichtung und/oder von dem Wischer durch Spülen und/oder Absaugen des Einführvolumens (106) abzulösen.

6. Mikrofluidikvorrichtung (100, 200A, 200B, 300, 602) nach einem der vorhergehenden Ansprüche, wobei das Messvolumen (110) einen ersten Messkanal und einen zweiten Messkanal, der parallel zu dem ersten Messkanal verbunden ist, umfasst, wobei der zweite Messkanal eine größere Querschnittsfläche als der erste Messkanal aufweist, insbesondere wobei die Mikrofluidikvorrichtung (100, 200A, 200B, 300, 602) eine Pumpe und/oder ein Ventil zum selektiven Leiten von Probenobjekten (102A-C) entweder in den ersten Messkanal oder den zweiten Messkanal umfasst und/oder wobei die Sortiereinheit (108) dazu eingerichtet ist, Probenobjekte (102A-C) basierend auf einer Größe, einem Gewicht und/oder einer Steifigkeit des jeweiligen Probenobjekts (102A-C) selektiv entweder in den ersten Messkanal oder den zweiten Messkanal zu leiten.

7. Mikrofluidikvorrichtung (100, 200A, 200B, 300, 602) nach einem der vorhergehenden Ansprüche, wobei:
die Mikrofluidikvorrichtung (100, 200A, 200B, 300, 602) ferner einen Messfluideinlass umfasst, der zwischen dem Auslass der Dissoziationseinheit (114) und dem Einlass des Messvolumens (110) angeordnet ist, um ein Messfluid zuzuführen; und/oder
das Messvolumen (110) ein optisches Detektionsfenster (112) zum Aufnehmen mikroskopischer Bilder, insbesondere Phasenverschiebungsbilder von Probenobjekten (102A-C) in dem Messvolumen (110), umfasst.

8. Verfahren (700) zum Analysieren einer Probe (102), die ein oder mehrere Probenobjekte (102A-C) umfasst, die biologische Zellen enthalten, unter Verwendung einer Mikrofluidikvorrichtung (100, 200A, 200B, 300, 602) nach einem der vorhergehenden Ansprüche, wobei das eine oder die mehreren Probenobjekte (102A-C) eine oder mehrere einzelne Zellen (102A), ein oder mehrere Zellaggregate (102B) und/oder ein oder mehrere Stücke von kontinuierlichem Gewebematerial (102C) umfassen, wobei das Verfahren (700) Folgendes umfasst:
Erzeugen eines Flusses eines Trägerfluids durch das Einführvolumen (106), um Probenobjekte (102A-C) von dem Einführvolumen (106) zu der Sortiereinheit (108) zu transportieren;
Sortieren der Probenobjekte (102A-C) mit der Sortiereinheit (108) in eine erste Charge von Probenobjekten und eine erste Restprobe, wobei die erste Restprobe Probenobjekte (102A-C) mit einer größeren Größe, einem größeren Gewicht und/oder einer höheren Steifigkeit als die Probenobjekte (102A-C) in der ersten Charge umfasst;
Durchführen einer ersten Messung an den Probenobjekten (102A-C) der ersten Charge in dem Messvolumen (110);
zumindest teilweises Dissoziieren von Probenobjekten (102A-C) in der ersten Restprobe mit der Dissoziationseinheit (114) in einzelne Zellen und/oder Zellaggregate, um eine zweite Charge von Probenobjekten zu erhalten; und
Durchführen einer zweiten Messung an den dissoziierten Probenobjekten (102A-C) in der zweiten Charge in dem Messvolumen (110).

9. Verfahren (700) nach Anspruch 8, wobei die zweite Charge von Probenobjekten durch Sortieren der dissoziierten Probenobjekte (102A-C) in die zweite Charge von Probenobjekten und eine zweite Restprobe erhalten wird, wobei die zweite Restprobe Probenobjekte (102A-C) mit einer größeren Größe, einem größeren Gewicht und/oder einer höheren Steifigkeit als die Probenobjekte (102A-C) in der zweiten Charge umfasst, wobei das Verfahren (700) vorzugsweise ferner das Erhalten einer oder mehrerer weiterer Chargen von Probenobjekten umfasst durch:
zumindest teilweises Dissoziieren von Probenobjekten (102A-C) in einer Restprobe, die von einer vorherigen Charge getrennt wurde, in einzelne Zellen und/oder Zellaggregate unter Verwendung der Dissoziationseinheit (114); und
Sortieren der dissoziierten Probenobjekte (102A-C) in die jeweilige weitere Charge von Probenobjekten und eine neue Restprobe, wobei die neue Restprobe Probenobjekte (102A-C) mit einer größeren Größe, einem größeren Gewicht und/oder einer höheren Steifigkeit als die Probenobjekte (102A-C) in der jeweiligen weiteren Charge umfasst.

10. Verfahren (700) nach Anspruch 8 oder 9, wobei:
das Verfahren ferner das Bestimmen eines Probentyps der Probe (102) umfasst, wobei der Probentyp eines oder mehrere von einem Verfahren, das zum Erhalten der Probe (102) von einem Patienten verwendet wird, einem Körperteil, aus dem die Probe (102) erhalten wurde, einer Größe der Probenobjekte (102A-C) in der Probe (102) und einem Gewicht der Probenobjekte (102A-C) in der Probe (102) charakterisiert; das Bestimmen einer Anzahl von Dissoziationsschritten, die durchzuführen sind, basierend auf dem Probentyp; und das Durchführen der Dissoziationsschritte und das Sortieren der dissoziierten Probenobjekte (102A-C) nach Größe, Gewicht und/oder Steifigkeit, um eine Vielzahl von Chargen von Probenobjekten zu erhalten; und/oder
die Probe (102) eine Biopsieprobe, eine Resektionsprobe, eine Aspirationsprobe, eine Körperflüssigkeitsprobe und/oder eine Abstrichprobe umfasst,
insbesondere wobei kein Dissoziationsschritt oder ein einzelner Dissoziationsschritt durchgeführt wird, wenn der Probentyp anzeigt, dass die Probe (102) eine Körperflüssigkeitsprobe ist, und/oder wenn der Probentyp anzeigt, dass die Probe (102) eine Abstrichprobe ist, und mehrere Dissoziationsschritte durchgeführt werden, wenn der Probentyp anzeigt, dass die Probe (102) eine Biopsieprobe ist, wenn der Probentyp anzeigt, dass die Probe eine Resektionsprobe ist, und/oder wenn der Probentyp anzeigt, dass die Probe (102) eine Aspirationsprobe ist.

11. Verfahren (700) nach einem der Ansprüche 8 bis 10, wobei:
die Dissoziationseinheit (114) der Mikrofluidikvorrichtung (100, 200A, 200B, 300, 602) eine Vielzahl von Mitteln (116, 116A-D) zum Dissoziieren der Probenobjekte (102A-C) umfasst und das Verfahren (700) ferner das Auswählen eines Mittels zum Dissoziieren der Probenobjekte aus der Vielzahl von Mitteln (116, 116A-D) zum Dissoziieren der Probenobjekte (102A-C) basierend auf dem Probentyp umfasst; und/oder
das Verfahren (700) ferner das Bestimmen einer Objektanzahl und/oder eines Zellviabilitätsmaßes für eine oder mehrere der Chargen von Probenobjekten durch optische, elektrische und/oder mechanische Mittel umfasst, insbesondere wobei die Objektanzahl für eine oder mehrere der Chargen bestimmt wird und das Verfahren (700) ferner das Anpassen einer Dichte von Probenobjekten (102A-C) in der jeweiligen Charge basierend auf der bestimmten Objektanzahl durch Zuführen eines Messfluids umfasst.

12. Verfahren (700) nach einem der Ansprüche 8 bis 11, wobei:
das Durchführen der ersten Messung und/oder das Durchführen der zweiten Messung das Aufnehmen eines Bildes der Probenobjekte (102A-C) in dem Messvolumen (110) unter Verwendung eines Mikroskops umfasst oder diesem entspricht, insbesondere wobei das Durchführen der ersten Messung und/oder das Durchführen der zweiten Messung das Aufnehmen eines Phasenverschiebungsbildes der Probenobjekte (102A-C) in dem Messvolumen (110) unter Verwendung eines Phasenkontrastmikroskops (608) umfasst oder diesem entspricht; und/oder
das Durchführen der ersten Messung und/oder das Durchführen der zweiten Messung das hydrodynamische und/oder viskoelastische Fokussieren der jeweiligen Probenobjekte (102A-C) in dem Messvolumen (110) umfasst.

13. System (600) zum Analysieren einer Probe (102), die ein oder mehrere Probenobjekte (102A-C) umfasst, die biologische Zellen enthalten, wobei das eine oder die mehreren Probenobjekte (102A-C) eine oder mehrere einzelne Zellen (102A), ein oder mehrere Zellaggregate (102B) und/oder ein oder mehrere Stücke von kontinuierlichem Gewebematerial (102C) umfassen, wobei das System (600) zur Verwendung mit einer Mikrofluidikvorrichtung (100, 200A, 200B, 300, 602) nach einem der Ansprüche 1 bis 7 eingerichtet ist und Folgendes umfasst:
eine Mikrofluidikeinheit (606), die dazu eingerichtet ist, einen Fluss eines Trägerfluids durch das Einführvolumen (106) der Mikrofluidikvorrichtung (100, 200A, 200B, 300, 602) zu erzeugen, um Probenobjekte (102A-C) von dem Einführvolumen (106) zu der Sortiereinheit (108) der Mikrofluidikvorrichtung (100, 200A, 200B, 300, 602) zu transportieren;
eine Messvorrichtung (608), die dazu eingerichtet ist, Messungen an einzelnen Zellen und/oder Zellaggregaten in dem Messvolumen (110) der Mikrofluidikvorrichtung (100, 200A, 200B, 300, 602) durchzuführen; und
eine Steuerung (610), die dazu eingerichtet ist, die Mikrofluidikeinheit (606), die Messvorrichtung (608), die Sortiereinheit (108) der Mikrofluidikvorrichtung (100, 200A, 200B, 300, 602) und/oder die Dissoziationseinheit (114) der Mikrofluidikvorrichtung (100, 200A, 200B, 300, 602) zu steuern, um ein Verfahren (700) nach einem der Ansprüche 8 bis 12 auszuführen.

14. System (600) nach Anspruch 13, wobei die Messvorrichtung (608) ein Phasenkontrastmikroskop, insbesondere ein digitales holographisches Mikroskop (608A-D), ist oder umfasst, das dazu eingerichtet ist, Phasenverschiebungsbilder von einzelnen Zellen und/oder Zellaggregaten in dem Messvolumen (110) der Mikrofluidikvorrichtung (100, 200A, 200B, 300, 602) aufzunehmen.

15. System (600) nach Anspruch 13 oder 14, wobei:
das System (600) ferner eine akustische Quelle (612), insbesondere eine Ultraschallquelle, umfasst, die dazu eingerichtet ist, akustische Wellen auf Probenobjekte (608A-D) in der Dissoziationseinheit (114) der Mikrofluidikvorrichtung (100, 200A, 200B, 300, 602) anzuwenden, wobei die Steuerung (610) dazu eingerichtet ist, die akustische Quelle (612) zu steuern, um Probenobjekte (102A-C) in der Dissoziationseinheit (114) zu dissoziieren, indem akustische Wellen auf die Probenobjekte (102A-C) angewendet werden; und/oder die Mikrofluidikeinheit (606) dazu eingerichtet ist, der Dissoziationseinheit (114) der Mikrofluidikvorrichtung (100, 200A, 200B, 300, 602) eine Auflösungslösung zuzuführen, wobei die Auflösungslösung eine oder mehrere chemische und/oder biochemische Substanzen zum chemischen Auflösen von Probenobjekten (102A-C) umfasst, insbesondere ein oder mehrere Enzyme zum Auflösen von Probenobjekten (102A-C) durch enzymatische Verdauung, wobei die Steuerung (610) dazu eingerichtet ist, die Mikrofluidikeinheit (606) zu steuern, um Probenobjekte (102A-C) in der Dissoziationseinheit (114) zu dissoziieren, indem die Auflösungslösung der Dissoziationseinheit (114) zugeführt wird; und/oder
das System (600) ferner eine Mikrofluidikvorrichtung (100, 200A, 200B, 300, 602) nach einem der Ansprüche 1 bis 7 umfasst.

## Revendications

1. Dispositif microfluidique (100, 200A, 200B, 300, 602) pour analyser un échantillon (102) comprenant un ou plusieurs objets échantillons (102A-C) contenant des cellules biologiques, dans lequel les un ou plusieurs objets échantillons (102A-C) comprennent une ou plusieurs cellules uniques (102A), un ou plusieurs agrégats de cellules (102B) et/ou un ou plusieurs morceaux de matériau tissulaire continu (102C), le dispositif microfluidique (100, 200A, 200B, 300, 602) comprenant :
un volume d'insertion (106) configuré pour recevoir l'échantillon (102) ;
une unité de tri (108) ayant une entrée qui est en communication fluidique avec le volume d'insertion (106), une première sortie (108A) et une deuxième sortie (108B), dans lequel l'unité de tri (108) est configurée pour trier les objets échantillons (102A-C) par taille, poids et/ou rigidité en dirigeant des objets échantillons (102A-C) ayant une taille moins grande, un poids moins grand et une rigidité inférieure, respectivement, de l'entrée vers la première sortie (108A) et des objets échantillons (102A-C) ayant une taille plus grande, un poids plus grand et une rigidité plus élevée, respectivement, de l'entrée vers la deuxième sortie (108B) ;
un volume de mesure (110) qui est en communication fluidique avec la première sortie (108A) de l'unité de tri (108) ; et
une unité de dissociation (114) comprenant des moyens (116, 116A-D) pour dissocier des objets échantillons (102A-C) en cellules uniques et/ou agrégats de cellules au moins en partie, dans lequel une entrée de l'unité de dissociation (114) est en communication fluidique avec la deuxième sortie (108B) de l'unité de tri (108) et une sortie de l'unité de dissociation (114) est en communication fluidique avec une entrée du volume de mesure (110).

2. Dispositif microfluidique (100, 200A, 200B, 300, 602) selon la revendication 1, dans lequel l'unité de dissociation (114) comprend un volume de dissociation (114A-D) et les moyens (116, 116A-D) de dissociation d'objets échantillons (102A-C) comprennent un ou plusieurs parmi :
un réservoir (116) configuré pour fournir une solution de dissolution au volume de dissociation (114A-D), la solution de dissolution comprenant une ou plusieurs substances chimiques et/ou biochimiques pour dissoudre chimiquement des objets échantillons (102A-C), en particulier un ou plusieurs enzymes pour dissoudre des objets échantillons (102A-C) par digestion enzymatique ;
un émetteur acoustique, en particulier un émetteur à ultrasons (116D), configuré pour appliquer des ondes acoustiques aux objets échantillons (102A-C) dans le volume de dissociation (114D) ;
un ou plusieurs obstacles statiques (116A) agencés dans le volume de dissociation (114A) configurés pour dissocier mécaniquement des objets échantillons (102A-C) entrant en collision avec les un ou plusieurs obstacles (116A) ;
une ou plusieurs portions incurvées et/ou rétrécies (116B) du volume de dissociation (114B) configurées pour dissocier des objets échantillons (102A-C) en générant une force de cisaillement sur des objets échantillons (102A-C) circulant à travers le volume de dissociation (114B) ;
un agitateur (116C) configuré pour déplacer une tige d'agitateur dans le volume de dissociation (114B) pour dissocier des objets échantillons (102A-C) ; et
un ou plusieurs éléments mobiles configurés pour couper et/ou broyer des objets échantillons (102A-C) dans le volume de dissociation (114A-D),
dans lequel de préférence l'unité de dissociation (114) comprend une pluralité de volumes de dissociation (114A-D), chacun d'eux étant associé à un moyen différent pour dissocier des objets échantillons, en particulier dans lequel l'unité de dissociation (114) comprend en outre une pluralité de pompes (118A-D) et/ou de soupapes (118A-D) pour diriger sélectivement des objets échantillons (102A-C) dans l'un des volumes de dissociation (114A-D) et/ou dans lequel l'unité de tri (108) est configurée pour diriger sélectivement des objets échantillons (102A-C) dans l'un des volumes de dissociation (114A-D) sur la base d'une taille, d'un poids et/ou d'une rigidité de l'objet échantillon (102A-C) respectif.

3. Dispositif microfluidique (100, 200A, 200B, 300, 602) selon la revendication 1 ou 2, dans lequel l'unité de tri (108) comprend un ou plusieurs parmi un filtre cellulaire basé sur l'inertie, un filtre cellulaire acoustique, un filtre cellulaire hydrodynamique, un filtre viscoélastique, un trieur de densité, un filtre de fractionnement par flux de champ, un filtre diélectrophorétique, un filtre à déplacement latéral déterministe, un filtre à déversoir, un filtre cellulaire en impasse, un trieur de cellules selon leur rigidité et un filtre cellulaire à flux transversal.

4. Dispositif microfluidique (100, 200A, 200B, 300, 602) selon l'une quelconque des revendications précédentes, dans lequel :
la sortie de l'unité de dissociation (114) est en communication fluidique avec le volume de mesure (110) via l'unité de tri (108) ; et/ou
la sortie de l'unité de dissociation (114) comprend en outre un trieur (202) ayant une première sortie (202A) qui est en communication fluidique avec le volume de mesure (110) et une deuxième sortie (202B) qui est en communication fluidique avec l'entrée de l'unité de dissociation (114), dans lequel le trieur (202) est configuré pour trier les objets échantillons (102A-C) par taille, poids et/ou rigidité en dirigeant des objets échantillons (102A-C) ayant une taille moins grande, un poids moins grand et une rigidité inférieure, respectivement, vers la première sortie (202A) et des objets échantillons (102A-C) ayant une taille plus grande, un poids plus grand et une rigidité supérieure, respectivement, vers la deuxième sortie (202B) ; et/ou
le dispositif microfluidique (100, 200A, 200B, 300, 602) comprend en outre un filtre (204) agencé entre la sortie de l'unité de dissociation (114) et l'entrée du volume de mesure (110), dans lequel le filtre (204) est configuré pour empêcher des objets échantillons (102A-C) ayant une taille plus grande qu'un seuil de filtrage de pénétrer dans le volume de mesure (110).

5. Dispositif microfluidique (100, 200A, 200B, 300, 602) selon l'une quelconque des revendications précédentes, dans lequel :
le dispositif microfluidique (100, 200A, 200B, 300, 602) comprend une unité de collecte d'échantillons (302) comprenant un ou plusieurs parmi un connecteur pour coupler une seringue et/ou un tube contenant un échantillon comprenant les un ou plusieurs objets échantillons (102A-C) au volume d'insertion (106) ; une ouverture configurée pour recevoir une aiguille d'aspiration et/ou une aiguille de biopsie, dans lequel l'ouverture est en communication fluidique avec le volume d'insertion (106) ; et une pompe de transfert configurée pour transférer un échantillon d'un tube de collecte vers le volume d'insertion (106) ; et/ou
le dispositif microfluidique (100, 200A, 200B, 300, 602) comprend en outre un dispositif d'essuyage pour essuyer un échantillon (102) d'un dispositif de collecte d'échantillons, en particulier pour essuyer un échantillon de biopsie d'une aiguille de biopsie ; et/ou le dispositif microfluidique (100, 200A, 200B, 300, 602) comprend en outre une pompe de circulation configurée pour générer une circulation à travers le volume d'insertion (106) pour transporter les objets échantillons (102A-C) du volume d'insertion (106) vers l'unité de tri (108), en particulier dans lequel la pompe de circulation est configurée pour détacher un échantillon (102) d'un dispositif de collecte d'échantillons et/ou du dispositif d'essuyage par rinçage et/ou aspiration du volume d'insertion (106).

6. Dispositif microfluidique (100, 200A, 200B, 300, 602) selon l'une quelconque des revendications précédentes, dans lequel le volume de mesure (110) comprend un premier canal de mesure et un deuxième canal de mesure connecté en parallèle au premier canal de mesure, le deuxième canal de mesure ayant une section transversale plus grande que le premier canal de mesure, en particulier dans lequel le dispositif microfluidique (100, 200A, 200B, 300, 602) comprend une pompe et/ou une soupape pour diriger sélectivement des objets échantillons (102A-C) dans soit le premier canal de mesure soit le deuxième canal de mesure et/ou dans lequel l'unité de tri (108) est configurée pour diriger sélectivement des objets échantillons (102A-C) dans soit le premier canal de mesure soit le deuxième canal de mesure sur la base d'une taille, d'un poids et/ou d'une rigidité de l'objet échantillon (102A-C) respectif.

7. Dispositif microfluidique (100, 200A, 200B, 300, 602) selon l'une quelconque des revendications précédentes, dans lequel :
le dispositif microfluidique (100, 200A, 200B, 300, 602) comprend en outre une entrée de fluide de mesure agencée entre la sortie de l'unité de dissociation (114) et l'entrée du volume de mesure (110) pour fournir un fluide de mesure ; et/ou
le volume de mesure (110) comprend une fenêtre de détection optique (112) pour prendre des images microscopiques, en particulier des images de déphasage d'objets échantillons (102A-C) dans le volume de mesure (110).

8. Procédé (700) d'analyse d'un échantillon (102) comprenant un ou plusieurs objets échantillons (102A-C) contenant des cellules biologiques à l'aide d'un dispositif microfluidique (100, 200A, 200B, 300, 602) selon l'une quelconque des revendications précédentes, dans lequel les un ou plusieurs objets échantillons (102A-C) comprennent une ou plusieurs cellules uniques (102A), un ou plusieurs agrégats de cellules (102B) et/ou un ou plusieurs morceaux de matériau tissulaire continu (102C), le procédé (700) comprenant :
la génération d'une circulation d'un fluide porteur à travers le volume d'insertion (106) pour transporter des objets échantillons (102A-C) du volume d'insertion (106) vers l'unité de tri (108) ;
le tri des objets échantillons (102A-C) avec l'unité de tri (108) en un premier lot d'objets échantillons et un premier échantillon résiduel, dans lequel le premier échantillon résiduel comprend des objets échantillons (102A-C) ayant une taille plus grande, un poids plus grand et/ou une rigidité plus élevée que les objets échantillons (102A-C) dans le premier lot ;
la réalisation d'une première mesure sur les objets échantillons (102A-C) du premier lot dans le volume de mesure (110) ;
la dissociation d'objets échantillons (102-C) dans le premier échantillon résiduel avec l'unité de dissociation (114) en cellules uniques et/ou agrégats de cellules au moins en partie pour obtenir un deuxième lot d'objets échantillons ; et
la réalisation d'une deuxième mesure sur les objets échantillons (102A-C) dissociés dans le deuxième lot du volume de mesure (110).

9. Procédé (700) selon la revendication 8, dans lequel le deuxième lot d'objets échantillons est obtenu en triant les objets échantillons (102A-C) dissociés en le deuxième lot d'objets échantillons et en un deuxième échantillon résiduel, dans lequel le deuxième échantillon résiduel comprend des objets échantillons (102A-C) ayant une taille plus grande, un poids plus grand et/ou une rigidité plus élevée que les objets échantillons (102A-C) dans le deuxième lot, dans lequel le procédé (700) comprend en outre de préférence l'obtention d'un ou plusieurs lots supplémentaires d'objets échantillons par :
dissociation d'objets échantillons (102A-C) dans un échantillon résiduel séparé d'un lot précédent en cellules uniques et/ou agrégats de cellules au moins en partie à l'aide de l'unité de dissociation (114) ; et
tri des objets échantillons (102A-C) dissociés en le lot supplémentaire respectif d'objets échantillons et en un nouvel échantillon résiduel, dans lequel le nouvel échantillon résiduel comprend des objets échantillons (102A-C) ayant une taille plus grande, un poids plus grand et/ou une rigidité plus élevée que les objets échantillons (102A-C) dans le lot supplémentaire respectif.

10. Procédé (700) selon la revendication 8 ou 9, dans lequel :
le procédé comprend en outre la détermination d'un type d'échantillon de l'échantillon (102), dans lequel le type d'échantillon caractérise un ou plusieurs parmi une procédure utilisée pour obtenir l'échantillon (102) en provenance d'un patient, une partie corporelle à partir de laquelle l'échantillon (102) a été obtenu, une taille d'objets échantillons (102A-C) dans l'échantillon (102) et un poids d'objets échantillons (102A-C) dans l'échantillon (102) ; la détermination d'un nombre d'étapes de dissociation qui doivent être réalisées sur la base du type d'échantillon ; et la réalisation des étapes de dissociation et le tri des objets échantillons (102A-C) dissociés par la taille, le poids et/ou la rigidité pour obtenir une pluralité de lots d'objets échantillons ; et/ou
l'échantillon (102) comprend un échantillon de biopsie, un échantillon de résection, un échantillon d'aspiration, un échantillon de fluide corporel et/ou un échantillon de frottis, en particulier dans lequel aucune étape de dissociation ou une étape de dissociation unique n'est/est réalisée si le type d'échantillon indique que l'échantillon (102) est un échantillon de fluide corporel et/ou si le type d'échantillon indique que l'échantillon (102) est un échantillon de frottis et de multiples étapes de dissociation sont réalisées si le type d'échantillon indique que l'échantillon (102) est un échantillon de biopsie, si le type d'échantillon indique que l'échantillon est un échantillon de résection et/ou si le type d'échantillon indique que l'échantillon (102) est un échantillon d'aspiration.

11. Procédé (700) selon l'une quelconque des revendications 8 à 10, dans lequel :
l'unité de dissociation (114) du dispositif microfluidique (100, 200A, 200B, 300, 602) comprend une pluralité de moyens (116, 116A-D) pour dissocier les objets échantillons (102A-C) et le procédé (700) comprend en outre la sélection d'un moyen pour dissocier les objets échantillons parmi une pluralité de moyens (116, 116A-D) pour dissocier les objets échantillons (102A-C) sur la base du type d'échantillon ; et/ou
le procédé (700) comprend en outre la détermination d'un comptage d'objets et/ou d'une mesure de viabilité cellulaire pour un ou plusieurs des lots d'objets échantillons par des moyens optiques, électriques et/ou mécaniques, en particulier dans lequel le comptage d'objets est déterminé pour un ou plusieurs des lots et le procédé (700) comprend en outre l'ajustement d'une densité d'objets échantillons (102A-C) dans le lot respectif sur la base du comptage d'objets déterminé en fournissant un fluide de mesure.

12. Procédé (700) selon l'une quelconque des revendications 8 à 11, dans lequel :
la réalisation de la première mesure et/ou la réalisation de la deuxième mesure comprend ou correspond à la prise d'une image des objets échantillons (102A-C) dans le volume de mesure (110) à l'aide d'un microscope, en particulier dans lequel la réalisation de la première mesure et/ou la réalisation de la deuxième mesure comprend ou correspond à la prise d'une image de déphasage des objets échantillons (102A-C) dans le volume de mesure (110) à l'aide d'un microscope à contraste de phase (608) ; et/ou
la réalisation de la première mesure et/ou la réalisation de la deuxième mesure comprend la focalisation hydrodynamique et/ou viscoélastique des objets échantillons (102A-C) respectifs dans le volume de mesure (110).

13. Système (600) d'analyse d'un échantillon (102) comprenant un ou plusieurs objets échantillons (102A-C) contenant des cellules biologiques, les un ou plusieurs objets échantillons (102A-C) comprenant une ou plusieurs cellules uniques (102A), un ou plusieurs agrégats de cellules (102B) et/ou un ou plusieurs morceaux de matériau tissulaire continu (102C), dans lequel le système (600) est configuré pour une utilisation avec le dispositif microfluidique (100, 200A, 200B, 300, 602) selon l'une quelconque des revendications 1 à 7 et comprend :
une unité microfluidique (606) configurée pour générer une circulation d'un fluide porteur à travers le volume d'insertion (106) du dispositif microfluidique (100, 200A, 200B, 300, 602) pour transporter des objets échantillons (102A-C) du volume d'insertion (106) vers l'unité de tri (108) du dispositif microfluidique (100, 200A, 200B, 300, 602) ;
un dispositif de mesure (608) configuré pour réaliser des mesures sur des cellules unique et/ou des agrégats de cellules dans le volume de mesure (110) du dispositif microfluidique (100, 200A, 200B, 300, 602) ; et
un dispositif de commande (610) configuré pour commander à l'unité microfluidique (606), au dispositif de mesure (608), à l'unité de tri (108) du dispositif microfluidique (100, 200A, 200B, 300, 602) et/ou à l'unité de dissociation (114) du dispositif microfluidique (100, 200A, 200B, 300, 602) d'exécuter un procédé (700) selon l'une quelconque des revendications 8 à 12.

14. Système (600) selon la revendication 13, dans lequel le dispositif de mesure (608) est ou comprend un microscope à contraste de phase, en particulier un microscope holographique numérique (608A-D), configuré pour prendre des images de déphasage de cellules uniques et/ou d'agrégats de cellules dans le volume de mesure (110) du dispositif microfluidique (100, 200A, 200B, 300, 602).

15. Système (600) selon la revendication 13 ou 14, dans lequel :
le système (600) comprend en outre une source acoustique (612), en particulier une source d'ultrasons, qui est configurée pour appliquer des ondes acoustiques aux objets échantillons (608A-D) dans l'unité de dissociation (114) du dispositif microfluidique (100, 200A, 200B, 300, 602), dans lequel le dispositif de commande (610) est configuré pour commander à la source acoustique (612) de dissocier des objets échantillons (102A-C) dans l'unité de dissociation (114) en appliquant des ondes acoustiques aux objets échantillons (102A-C) ; et/ou
l'unité microfluidique (606) est configurée pour fournir une solution de dissolution à l'unité de dissociation (114) du dispositif microfluidique (100, 200A, 200B, 300, 602), la solution de dissolution comprenant une ou plusieurs substances chimiques et/ou biochimiques pour dissoudre chimiquement des objets échantillons (102A-C), en particulier un ou plusieurs enzymes pour dissoudre des objets échantillons (102A-C) par digestion enzymatique, dans lequel le dispositif de commande (610) est configuré pour commander à l'unité microfluidique (606) de dissocier des objets échantillons (102A-C) dans l'unité de dissociation (114) en fournissant la solution de dissolution à l'unité de dissociation (114) ; et/ou
le système (600) comprend en outre un dispositif microfluidique (100, 200A, 200B, 300, 602) selon l'une quelconque des revendications 1 à 7.
